# EUROPEAN PATENT APPLICATION

(11) **EP 4 361 287 A2**
(43) Date of publication of application: **01.05.2024**
(21) Application number: 24162689.4
(22) Date of filing: 28.09.2017
(51) Int. Cl.: C12Q 1/6869

(54) **METHODS FOR SEQUENCING NUCLEIC ACIDS USING TERMINATION CHEMISTRY**

(30) Priority: 28.09.2016 US 201662400681 P; 28.09.2016 US 201662400693 P
(62) Divisional of application: 17781331.8
(71) Applicant: Life Technologies Corporation, Carlsbad, CA 92008 (US)
(72) Inventor: HUBBELL, Earl, Palo Alto (US)
(74) Representative: Thermo Fisher Scientific- Life Sciences Solutions Group

(57) **Abstract**

A method for nucleic acid sequencing may include disposing a plurality of template nucleic acid molecules in a plurality of defined spaces disposed on a sensor array, at least some of the plurality of template nucleic acid molecules having a sequencing primer and a polymerase operably bound therewith; advancing one or more nucleotide species over the plurality of template nucleic acid molecules with the sequencing primer and the polymerase operably bound therewith; measuring a signal generated by nucleotide incorporations resulting from advancing the one or more nucleotide species; and exposing the plurality of template nucleic acid molecules to a cleaving reagent subsequent to the advancing and measuring. The cleaving reagent can remove labeling reagents attached to the one or more nucleotide species. The advancing and measuring steps can be performed for different orders of the one or more nucleotide species prior to a subsequent exposing of the plurality of template nucleic acid molecules to the cleaving reagent.

## Description

### CROSS REFERENCE TO RELATED APPLICATIONS

This application claims priority to U.S. Prov. Appl. No. 62/400,693, filed September 28, 2016, and to U.S. Prov. Appl. No. 62/400,681, filed on September 28, 2016, each of which is incorporated by reference herein in its entirety.

### TECHNICAL FIELD

This application generally relates to methods, systems, and computer readable media for nucleic acid sequencing, and, more particularly, to methods, systems, and computer readable media for reducing phasing errors in nucleic acid sequencing.

### BACKGROUND

Nucleic acid sequencing, in which the order of nucleotides (including adenosine, guanosine, cytosine, thymidine, and uridine) in a nucleic acid molecule is determined, has become ubiquitous in a wide variety of medical applications, such as biological research, genetic testing, and so forth. One type of sequencing utilized in such applications is sequencing-by-synthesis in which the order of nucleotides in a nucleic acid strand is determined by synthesizing a corresponding strand. Sequencing-by-synthesis is a high throughput method employed in many platforms including but not limited to, for example, the Genome Analyzer/HiSeq/MiSeq platforms (Illumina, Inc.; see, e.g., U.S. Pat. Nos. 6,833,246 and 5,750,341); the GS FLX, GS FLX Titanium, and GS Junior platforms (Roche/454 Life Sciences; see, e.g., Ronaghi et al., SCIENCE, 281:363-365 (1998), and Margulies et al., NATURE, 437:376-380 (2005)); and the Ion Personal Genome Machine (PGM.TM.) and Ion Proton.TM. (Life Technologies Corp./Ion Torrent; see, e.g., U.S. Pat. No. 7,948,015 and U.S. Pat. Appl. Publ. Nos. 2010/0137143, 2009/0026082, and 2010/0282617, which are all incorporated by reference herein in their entirety).

Sequencing-by-synthesis and other platforms generate large volumes of sequencing data that must subsequently be processed to determine the order of the nucleotides in a given nucleic acid strand. Various sources of errors can impact the accuracy of sequencing data obtained via these methods. Such sources include, for example, loss of phase synchrony (*i.e*., loss of synchronous synthesis of the identical templates), that hinder the ability to make accurate base calls. Accordingly, there exists a need for improvement of systems and methods that perform sequencing while reducing or minimizing sequencing errors associated with various phase loss effects that may occur with sequencing-by-synthesis, and enable more accurate and efficient handling of the large volumes of sequencing data obtained via the sequencing-by-synthesis platforms. In addition, it is desirable to provide sequencing techniques that can accurately identify the sequences of relatively long sequences and/or homopolymers.

### SUMMARY

Exemplary embodiments of the present disclosure may solve one or more of the above-mentioned problems and/or may demonstrate one or more of the above-mentioned desirable features. Other features and/or advantages may become apparent from the description that follows.

In accordance with at least one exemplary embodiment, the present disclosure contemplates a method for nucleic acid sequencing, the method including disposing a plurality of template nucleic acid molecules in a plurality of defined spaces disposed on a sensor array, at least some of the plurality of template nucleic acid molecules having a sequencing primer and a polymerase operably bound therewith, advancing one or more nucleotide species over the plurality of template nucleic acid molecules with the sequencing primer and the polymerase operably bound therewith, measuring a signal generated by nucleotide incorporations resulting from advancing the one or more nucleotide species, and exposing the plurality of template nucleic acid molecules to a cleaving reagent subsequent to the advancing and measuring. The cleaving reagent removes labeling reagents attached to the one or more nucleotide species. The advancing and measuring steps may be performed for different orders of the one or more nucleotide species prior to a subsequent exposing of the plurality of template nucleic acid molecules to the cleaving reagent.

In a related exemplary embodiment, the exposing of the plurality of template nucleic acid molecules to the cleaving reagent occurs subsequent to the advancing and measuring for each individual nucleotide species.

In another related exemplary embodiment, the exposing occurs subsequent to the advancing and measuring for a pair of nucleotide species. The advancing and measuring steps may be repeated for different orders of nucleotide species per pair of nucleotide species prior to subsequent exposing steps.

In another related exemplary embodiment, the exposing occurs subsequent to performing the advancing and measuring for a triplet of nucleotide species. The advancing and measuring steps are repeated for different orders of nucleotide species per triplet of nucleotide species prior to subsequent exposing steps. The method may further be repeated for alternating combinations of nucleotide species per triplet of nucleotide species.

In another related exemplary embodiment, the exposing occurs subsequent to performing the advancing and measuring for a quad of nucleotide species. The advancing and measuring steps may be repeated for different orders of nucleotide species per quad of nucleotide species prior to subsequent exposing steps. The method may be repeated for alternating combinations of nucleotide species per quad of nucleotide species.

In another related exemplary embodiment, the advancing comprises advancing a first nucleotide species over the plurality of template nucleic acid molecules, and the measuring comprises measuring a signal generated by nucleotide incorporations resulting from advancing the first nucleotide species. In this embodiment, the method further includes subsequently advancing a second nucleotide species over the plurality of template nucleic acid molecules, and measuring a signal generated by nucleotide incorporations resulting from advancing the second nucleotide species. The method further includes exposing the plurality of template nucleic acid molecules to the cleaving reagent prior to subsequently advancing the second nucleotide species, wherein the cleaving reagent removes a first labeling reagent attached to the first nucleotide species. The method may further include exposing the plurality of template nucleic acid molecules to the cleaving reagent subsequent to measuring the signal generated by nucleotide incorporations resulting from advancing the second nucleotide species, wherein the cleaving reagent removes a second labeling reagent attached to the second nucleotide species.

In this related embodiment, the method may further include advancing a third nucleotide species over the plurality of template nucleic acid molecules, measuring a signal generated by nucleotide incorporations resulting from advancing the third nucleotide species, subsequently advancing a fourth nucleotide species over the plurality of template nucleic acid molecules, and measuring a signal generated by nucleotide incorporations resulting from advancing the fourth nucleotide species. The fourth nucleotide species may be the same as one of the first, second, or third nucleotide species. The method may further include exposing the plurality of template nucleic acid molecules to the cleaving reagent subsequent to measuring the signal generated by nucleotide incorporations resulting from advancing the second nucleotide species and prior to advancing the third nucleotide species, wherein the cleaving reagent removes labeling reagents attached to the first and second nucleotide species.

The method may further include exposing the plurality of template nucleic acid molecules to the cleaving reagent subsequent to measuring the signal generated by nucleotide incorporations resulting from advancing the third nucleotide species and prior to advancing the fourth nucleotide species, wherein the cleaving reagent removes labeling reagents attached to the first, second, and third nucleotide species.

The method may further include exposing the plurality of template nucleic acid molecules to the cleaving reagent subsequent to measuring the signal generated by nucleotide incorporations resulting from advancing the fourth nucleotide species and prior to advancing a fifth nucleotide species, wherein the cleaving reagent removes labeling reagents attached to the first, second, third, and fourth nucleotide species, and wherein the fifth nucleotide species comprises any one of the first, second, third, or fourth nucleotide species.

In exemplary embodiments, each of the methods described herein may further include re-advancing at least one of the one or more nucleotide species over the plurality of template nucleic acid molecules in a smaller concentration and for a shorter duration than the advancing of said at least one nucleotide species. Different combinations/orders of the nucleotide species may be advanced and measured any number of times prior to performing the re-advancing.

In accordance with at least another exemplary embodiment, the present disclosure contemplates a method for nucleic acid sequencing, including disposing a plurality of template nucleic acid molecules in a plurality of defined spaces disposed on a sensor array, at least some of the plurality of template nucleic acid molecules having a sequencing primer and a polymerase operably bound therewith, advancing a mixture of nucleotide species over the plurality of template nucleic acid molecules with the sequencing primer and the polymerase operably bound therewith, measuring a signal generated by advancing the mixture of nucleotide species, and cleaving a labeling reagent from one or more of the mixture of nucleotide species. The advancing of the mixture of nucleotides species and measuring signals generated therefrom may be performed for different orders of mixture of nucleotide species prior to a subsequent cleaving.

In a related exemplary embodiment, measuring the signal comprises measuring a cumulative signal generated by nucleotide incorporations resulting from advancing the mixture nucleotide species, and determining a contribution to the cumulative signal of each nucleotide species in the mixture of nucleotide species. Further, the mixture of nucleotide species may be advanced in a phase-protecting flow order.

In accordance with at least another exemplary embodiment, the subject disclosure contemplates a method for nucleic acid sequencing, including disposing a plurality of template nucleic acid molecules in a plurality of defined spaces disposed on a sensor array, at least some of the plurality of template nucleic acid molecules having a sequencing primer and a polymerase operably bound therewith, advancing a first pair of nucleotide species over the plurality of template nucleic acid molecules with the sequencing primer and the polymerase operably bound therewith, each of the first pair of nucleotide species being labeled with a first labeling reagent, measuring a first signal generated by nucleotide incorporations resulting from advancing the first pair of nucleotide species, exposing the plurality of template nucleic acid molecules to a cleaving reagent, wherein the cleaving reagent removes the first labeling reagent attached to a first nucleotide species of the first pair of nucleotide species, and measuring a second signal generated by nucleotide incorporations resulting from a second nucleotide species of the first pair of nucleotide species labeled with the first labeling reagent. The cleaving agent removes the first labeling reagent attached to the first nucleotide species by removing a first linker molecule. The method further includes exposing the plurality of template nucleic acid molecules to a cleaving reagent, wherein the cleaving reagent removes the first labeling reagent attached to a second nucleotide species of the first pair of nucleotide species.

Additional objects, features, and/or advantages will be set forth in part in the description which follows, and in part will be obvious from the description, or may be learned by practice of the present disclosure and/or claims. At least some of these objects and advantages may be realized and attained by the elements and combinations particularly pointed out in the appended claims.

It is to be understood that both the foregoing general description and the following detailed description are exemplary and explanatory only and are not restrictive of the claims; rather the claims should be entitled to their full breadth of scope, including equivalents.

### BRIEF DESCRIPTION OF THE DRAWINGS

The present disclosure can be understood from the following detailed description, either alone or together with the accompanying drawings. The drawings are included to provide a further understanding of the present disclosure, and are incorporated in and constitute a part of this specification. The drawings illustrate one or more exemplary embodiments of the present teachings and together with the description serve to explain certain principles and operation.
FIG. 1 is a schematic illustration of a system for identifying a nucleic acid sequence, according to an exemplary embodiment of the present disclosure.
FIG. 2A is a schematic illustration of a simulation framework for calculating predicted ionograms, according to an exemplary embodiment of the present disclosure.
FIG. 2B illustrates an example cell within the simulation framework of FIG. 2A along with possible states and state transitions, according to an exemplary embodiment of the present disclosure.
FIG. 3A is a schematic representation of various sequencing reaction steps, according to an exemplary embodiment of the present disclosure.
FIG. 3B is a flow chart illustrating a workflow corresponding to the schematic representation of FIG. 3A.
FIG. 4 illustrates exemplary simulation data corresponding to signal response curves for the sequencing reaction steps of FIG. 3A.
FIGS. 5A-5D illustrate exemplary simulation data corresponding to template population evolution as sequencing progresses for the sequencing reaction steps of FIG. 3A.
FIGS. 6A-6D illustrate exemplary simulation data corresponding to partially base-called simulated sequences for the sequencing reaction steps of FIG. 3A.
FIGS. 7A-7B are schematic representations of various sequencing reaction steps, according to other exemplary embodiments of the present disclosure.
FIG. 7C is a flow chart illustrating a workflow corresponding to the schematic representations of FIGS. 7A-7B.
FIG. 8 illustrates exemplary simulation data corresponding to signal response curves for the sequencing reaction steps of FIG. 7A.
FIGS. 9A-9D illustrate exemplary simulation data corresponding to template population evolution as sequencing progresses for the sequencing reaction steps of FIG. 7A.
FIGS. 10A-10D illustrate exemplary simulation data corresponding to partially base-called simulated sequences for the sequencing reaction steps of FIG. 7A.
FIG. 11 illustrates exemplary simulation data corresponding to signal response curves for the sequencing reaction steps of FIG. 7B.
FIGS. 12A-12B are schematic representations of various sequencing reaction steps, according to yet other exemplary embodiments of the present disclosure.
FIG. 12C is a flow chart illustrating a workflow corresponding to the schematic representations of FIGS. 12A-12B.
FIG. 13 illustrates exemplary simulation data corresponding to signal response curves for the sequencing reaction steps of FIG. 12A.
FIGS. 14A-14D illustrate exemplary simulation data corresponding to template population evolution as sequencing progresses for the sequencing reaction steps of FIG. 12A.
FIGS. 15A-15D illustrate exemplary simulation data corresponding to partially base-called simulated sequences for the sequencing reaction steps of FIG. 12A.
FIG. 16 illustrates exemplary simulation data corresponding to signal response curves for the sequencing reaction steps of FIG. 12B.
FIG. 17Ais a schematic representation of various sequencing reaction steps, according to yet another exemplary embodiment of the present disclosure.
FIG. 17B is a flow chart illustrating a method for performing a nucleotide flow based on the schematic representation of FIG. 17A.
FIG. 18 illustrates exemplary simulation data corresponding to signal response curves for the sequencing reaction steps of FIG. 17A.
FIGS. 19A-19D illustrate exemplary simulation data corresponding to template population evolution as sequencing progresses for the sequencing reaction steps of FIG. 17A.
FIGS. 20A-20D illustrate exemplary simulation data corresponding to partially base-called simulated sequences for the sequencing reaction steps of FIG. 17A.
FIG. 21 is a schematic view of a system for identifying a nucleic acid sequence, according to another exemplary embodiment of the present disclosure.
FIGS. 22-26 are flow charts illustrating workflows for performing various different sequencing reaction steps, according to various exemplary embodiments of the present disclosure.

### DETAILED DESCRIPTION

This description and the accompanying drawings that illustrate exemplary embodiments should not be taken as limiting. Various mechanical, compositional, structural, electrical, and operational changes may be made without departing from the scope of this description and claims, including equivalents. In some instances, well-known structures and techniques have not been shown or described in detail so as not to obscure the disclosure. Like numbers in two or more figures represent the same or similar elements. Furthermore, elements and their associated features that are described in detail with reference to one embodiment may, whenever practical, be included in other embodiments in which they are not specifically shown or described. For example, if an element is described in detail with reference to one embodiment and is not described with reference to a second embodiment, the element may nevertheless be claimed as included in the second embodiment.

For the purposes of this specification and appended claims, unless otherwise indicated, all numbers expressing quantities, percentages, or proportions, and other numerical values used in the specification and claims, are to be understood as being modified in all instances by the term "about," to the extent they are not already so modified. Accordingly, unless indicated to the contrary, the numerical parameters set forth in the following specification and attached claims are approximations that may vary depending upon the desired properties sought to be obtained. At the very least, and not as an attempt to limit the application of the doctrine of equivalents to the scope of the claims, each numerical parameter should at least be construed in light of the number of reported significant digits and by applying ordinary rounding techniques.

It is noted that, as used in this specification and the appended claims, the singular forms "a," "an," and "the," and any singular use of any word, include plural referents unless expressly and unequivocally limited to one referent. As used herein, the term "include" and its grammatical variants are intended to be non-limiting, such that recitation of items in a list is not to the exclusion of other like items that can be substituted or added to the listed items.

As used herein, the term "nucleotide" and its variants refer to any compound that can bind selectively to, or can be polymerized by, a polymerase. Typically, but not necessarily, selective binding of the nucleotide to the polymerase is followed by polymerization of the nucleotide into a nucleic acid strand by the polymerase. Such nucleotides include not only naturally-occurring nucleotides but also any modified nucleotides or derivatives that, regardless of their structure, can bind selectively to and can optionally be polymerized by, a polymerase. While naturally-occurring nucleotides typically comprise sugar, base, and phosphate moieties, the modified nucleotides can include compounds lacking any one, some or all of such moieties, or can include one or more substitute groups.

As used herein, the term "polymerase" and its variants comprise any enzyme that can catalyze the polymerization of nucleotides (including blocked or reversibly blocked nucleotides including but not limited to 2' or 3' or 4' reversibly blocked nucleotides) into a nucleic acid strand. Typically but not necessarily such nucleotide polymerization can occur in a template-dependent fashion. Such polymerases can include without limitation naturally occurring polymerases and any subunits and truncations thereof, mutant polymerases, variant polymerases, recombinant, fusion, chimeric or otherwise engineered polymerases, chemically modified polymerases, synthetic molecules or assemblies, and any analogs, homologs, derivatives or fragments thereof that retain the ability to catalyze such polymerization. Optionally, the polymerase can be a mutant polymerase comprising one or more mutations involving the replacement of one or more amino acids with other amino acids, the insertion or deletion of one or more amino acids from the polymerase, or the linkage of parts, domains, or motifs of two or more polymerases. Typically, the polymerase comprises one or more active sites at which nucleotide binding and/or catalysis of nucleotide polymerization can occur. Some exemplary polymerases include without limitation DNA polymerases (such as for example Phi-29 DNA polymerase, reverse transcriptases and E. coli DNA polymerase) and RNA polymerases. The term "polymerase" and its variants, as used herein, also refers to fusion proteins comprising at least two portions linked to each other, where the first portion comprises a peptide that can catalyze the polymerization of nucleotides into a nucleic acid strand and is linked to a second portion that comprises a second polypeptide. In some embodiments, the second polypeptide can include a processivity-enhancing domain.

As used herein, the term "nucleotide incorporation" and its variants comprise polymerization of one or more nucleotides to form a nucleic acid strand including at least two nucleotides linked to each other, typically but not necessarily via phosphodiester bonds, although alternative linkages may be possible in the context of particular nucleotide analogs. In some embodiments, polymerization of the one or more nucleotides can include polymerization of a blocked or reversibly blocked nucleotide, including but not limited to, a 2' or 3' or 4' reversibly blocked nucleotide to a second nucleotide. Optionally, the second nucleotide is a blocked or reversibly blocked nucleotide.

Various exemplary embodiments disclosed herein are related to providing nucleotide flows and sequencing reaction steps that are designed to expedite sequencing procedures to maximize throughput, including the length of sequences that can be identified and sequences with homopolymers, while minimizing phase loss effects (hereinafter referred to as "phase effects" or "phase errors"). Generally, nucleotide flows described herein include one or more of the following steps that are performed in any order: an advancing step, a labeling step, a measuring step, a finishing step, a reset step, a cleave step, and a wash step. The systems and methods described herein incorporate various pre-determined nucleotide flow "orders" of these various steps designed to maximize throughput and minimize phase effects. For example, while measurement and advance steps are relatively fast, reset and cleave steps are relatively slow. Thus, exemplary sequencing reaction steps described herein minimize the occurrence or frequency of reset and cleave steps. Further, the described sequencing reaction steps reduce or eliminate the likelihood that incorrect bases are called due to the phasing effects, thereby reducing errors and improving the accuracy of sequencing.

Exemplary sequencing reaction steps described herein include advancing one or more terminating nucleotides in a series of flows to react with the nucleic acid sequence of interest, and measuring signals generated from the resulting incorporations of the individual types of nucleotides flowed. For example, sequencing reaction steps described herein include advance, measure, finish, and cleave/reset steps for a single terminating nucleotide, advance and measure steps for two different terminating nucleotides for every finish and cleave/reset step, advance and measure steps for three different terminating nucleotides for every finish and cleave/reset step, and advance and measure steps for four different terminating nucleotides for every finish and cleave/reset step. These and other features of various exemplary embodiments are discussed in more detail below with reference to the drawings. In addition, those having ordinary skill in the art would understand that other flow orders and sequencing reaction steps may be implemented to achieve similar results based on the principles described herein.

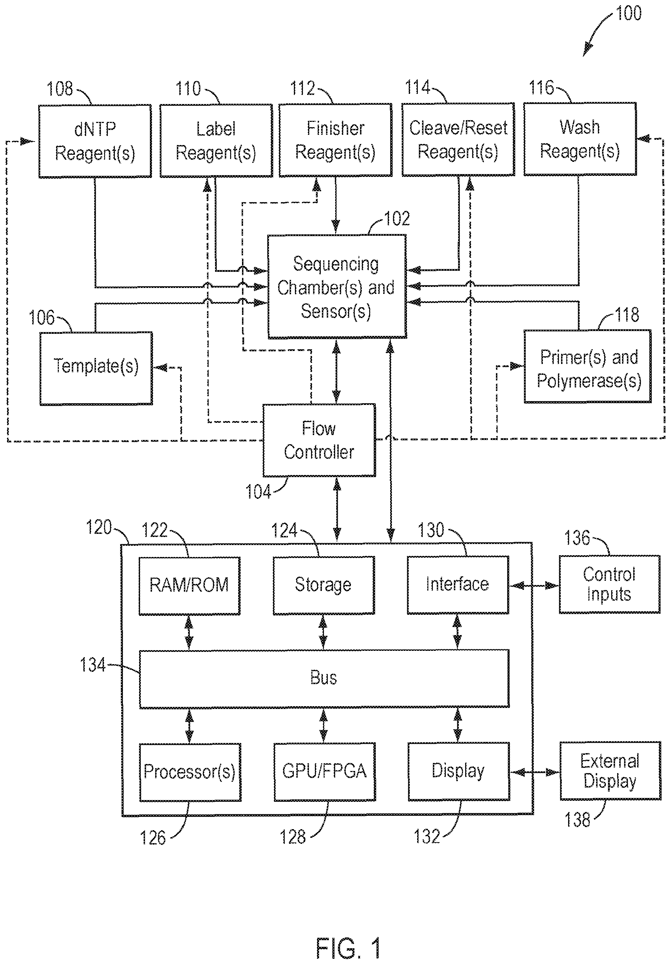

FIG. 1 illustrates components of an exemplary system 100 for nucleic acid sequencing. The components include a sequencing chamber 102, a flow controller 104, one or more template nucleic acids 106, one or more nucleotide flow reagents 108 comprising deoxynucleoside triphosphates (dNTPs), one or more label reagents 110, one or more finisher reagents 112, one or more cleave/reset reagents 114, one or more wash reagents 116, one or more primers and/or polymerases 118. System 100 further comprises a computing device 120 that includes memory 122, storage 124, one or more processors 126, graphics processing unit (GPU) 128, interface 130, and display 132 interconnected via bus 134, as well as control inputs 136 and external display 138.

As described herein, system 100 is configured to perform a sequencing-by-synthesis process using termination chemistry ("termination sequencing-by-synthesis"). As used herein, the term "termination sequencing-by-synthesis" encompasses all sequencing-by-synthesis processes that employ any type of termination chemistry. For example, termination sequencing-by-synthesis includes, but is not limited to, sequencing-by-synthesis processes in which nucleic acid replication is reversibly or irreversibly terminated in a stepwise fashion via incorporation of one or more terminators, such as chemically altered dNTPs (*e*.*g*., chemically altered dATP, dCTP, dGTP, and/or dTTP), including 2',3' dideoxynucleotides (ddNTPs) (*e*.*g*., ddATP, ddCTP, ddGTP, ddTTP) into the reaction mixture. In an exemplary embodiment utilizing electronic or charged-based sequencing (*e*.*g*., pH-based sequencing) employing termination chemistry, an incorporation signal generated from a nucleotide incorporation event within sequencing chamber 102 may be determined by detecting ions (*e*.*g*., hydrogen ions) that are generated as natural by-products of polymerase-catalyzed nucleotide extension reactions. This may be used to sequence a sample or template nucleic acid 106, which may be a fragment of a nucleic acid sequence of interest, for example, and which may be directly or indirectly attached as a clonal population to a solid support, such as a particle, microparticle, bead, etc. The sample or template nucleic acid 106 may be operably associated to a primer and/or polymerase 118. The template nucleic acid 106 may be subjected to repeated cycles or nucleotide flows or various reagents 108-116, from which nucleotide incorporations may result with corresponding generation of incorporation signals. Further, as understood by those of ordinary skill in the art, the particular type, mixture, and timing of the reactants provided to sequencing chamber 102 will vary depending on a variety of implementation-specific considerations, such as the type of sequencing-by-synthesis method being employed, the type of termination chemistry used, the available imaging or sensing platforms, and so forth. Accordingly, reagents 108-116 are non-limiting examples of the types of reactants that could be provided to the sequencing chamber 102. Further, exemplary embodiments disclosed herein provide various nucleotide flows or sequencing reaction steps that are designed to maximize throughput while minimizing phase errors.

In an exemplary embodiment, the primer-template-polymerase complex may be subjected to a series of exposures of different nucleotides in a pre-determined sequence or ordering. If one or more nucleotides are incorporated, then the signal resulting from the incorporation reaction may be detected, and after repeated cycles of nucleotide addition, primer extension, and signal acquisition, the nucleotide sequence of the template strand may be determined. The output signals measured throughout this process depend on the number of nucleotide incorporations. Specifically, in each addition step, the polymerase extends the primer by incorporating added dNTP only if the next base in the template is complementary to the added dNTP. If there is one complementary base, there is one incorporation; if two, there are two incorporations; if three, there are three incorporations, and so on. With each incorporation, an hydrogen ion is released, and collectively a population of released hydrogen ions changes the local pH of the reaction chamber. The production of hydrogen ions may be monotonically related to the number of contiguous complementary bases in the template (as well as to the total number of template molecules with primer and polymerase that participate in an extension reaction). Thus, when there is a number of contiguous identical complementary bases in the template (which may represent a homopolymer region), the number of hydrogen ions generated and thus the magnitude of the local pH change is proportional to the number of contiguous identical complementary bases (and the corresponding output signals are then sometimes referred to as "1-mer," "2-mer," "3-mer" output signals, etc.). If the next base in the template is not complementary to the added dNTP, then no incorporation occurs and no hydrogen ion is released (and the output signal is then sometimes referred to as a "0-mer" output signal).

In an exemplary embodiment, the terminator provided to the sequencing chamber 102 may include any of a variety of classes of terminators suitable for terminating primer extension. For example, suitable terminators include irreversible terminators, such as ddNTPs that lack a 3' hydroxyl and, thus, interrupt nucleic replication by virtue of a hydrogen instead of a hydroxyl at the 3' position. As an additional example, reversible terminators also may be utilized. Such terminators may include 3'-O-blocked reversible terminators and 3'-unblocked reversible terminators. Suitable 3'-O-blocked reversible terminators may include a terminating group linked to the oxygen atom of the 3' hydroxyl of the pentose. Several commercially available terminators of this type may be utilized in different implementations, including but not limited to 3'-ONH₂ reversible terminators, 3'-O-allyl reversible terminators, and 3'-O-azidomethy reversible terminators. Suitable 3'-unblocked reversible terminators include an intact 3' hydroxyl group and a terminating group linked to the base for termination of primer extension. Several commercially available terminators of this type may be utilized in different implementations, including but not limited to the 3'-OH unblocked reversible terminator named "virtual terminator" and the 3'-OH unblocked nucleotides termed "Lightening Terminators^{™}," which have a terminating 2-nitrobenzyl moiety attached to hydroxymethylated nucleobases. Depending on the type of terminator selected, the particular polymerase 118 selected for use in the processes performed by system 100 may vary. That is, the type of nucleotide analog selected for the nucleic acid sequencing may impact the type of DNA polymerase 118 that will yield the optimal efficiency. For example, in one embodiment, the Lightening Terminators^{™} may be selected for use as the terminator, and the Therminator^{™} DNA polymerase developed for use with the Lightening Terminators^{™} may be utilized to optimize efficiency. Additional details related to terminator chemistry are provided in International Application No. PCT / US 2016/023139, the contents of which are incorporated by reference herein in their entirety.

In other exemplary embodiments, template nucleotides 106 (including polynucleotides) may be sequenced using any sequencing technique, including sequencing-by-synthesis, ion-based sequencing involving the detection of sequencing byproducts using field effect transistors (e.g., FETs and ISFETs), chemical degradation sequencing, ligation-based sequencing, hybridization sequencing, pyrophosphate detection sequencing, capillary electrophoresis, gel electrophoresis, next-generation, massively parallel sequencing platforms, sequencing platforms that detect hydrogen ions or other sequencing by-products, and single molecule sequencing platforms. In some embodiments, a sequencing reaction can be conducted using at least one sequencing primer 118 that can hybridize to any portion of the nucleic acid template 106, including a nucleic acid adaptor or a target polynucleotide.

In an exemplary embodiment, sequencing chamber 102 includes a sensor array and/or a microwell array. For example, sequencing chamber 102 may include a flow path of reagents 108-116 over a combination of template nucleic acids 106 and primers/polymerases 118 within each microwell of the microwell array. In an exemplary embodiment, the microwell array may include an array of defined spaces or reaction confinement regions, such as microwells, for example, that is operationally associated with a sensor array so that, for example, each microwell has a sensor suitable for detecting an analyte or reaction property of interest. The microwell array may be integrated with the sensor array as a single device or chip within sequencing chamber 102. Sequencing chamber 102 may thus comprise a variety of designs for controlling the path and flow rate of reagents 108-116 over the microwell array. In an exemplary embodiment, sequencing chamber 102 comprises a microfluidics device.

Flow controller 104 (also referred to as a fluidics controller) may control the flow of the reagents 108-116 to sequencing chamber 102 (which may also be referred to herein as a reaction chamber). In various embodiments, the flow controller 104 may be configured (or programmed by computing device 120) to control driving forces for flowing reagents 108-116, template nucleic acids 106, and primers/polymerases 118 with any suitable instrument control software, such as LabView (National Instruments, Austin, Tex.), to deliver reagents 108-116 to sequencing chamber 102 according to a predetermined reagent flow ordering. The reagents 108-116 may be delivered for predetermined durations, at predetermined flow rates, and may measure physical and/or chemical parameters providing information about the status of one or more reactions taking place in defined spaces or reaction confinement regions, such as, for example, microwells. The reagents 110, 112, 114, and 116 may be driven through various fluid pathways, valves, and sequencing chamber 102 by pumps, gas pressure, or other suitable methods, and may be discarded after exiting the sequencing chamber 102. For example, system 100 may include various tubes for advancement of solutions, tubes for measurement, resetting and cleaving, inlets, outlets, valves, lines, passages, waste containers, electrodes, array controllers, etc. that are not depicted herein but will be apparent to those having ordinary skill in the art in light of this disclosure. Thus, the various combinations of sequencing reaction steps proposed herein may be implemented on any such instrument without being limited by the hardware features.

System 100 further includes a computing device 120 that receives nucleic acid sequencing data from sequencing chamber 102 for analysis and/or processing. Computing device 120 further comprises an internal bus 134 to which one or more processors 126 are connected to enable communication with a variety of other system components. For example, computing device 120 includes a memory 122 coupled to bus 134 for storing instructions to be executed by the one or more processors 126. Memory 122 may also be used for storing temporary variables or other intermediate information during execution of instructions to be executed by the one or more processors 126. Further, a storage device 124 is provided for storing static information and instructions for the one or more processors 126. Storage device 124 may include a magnetic disk, optical disk, or solid state drive (SSD) for storing information or instructions. Storage device 124 may further include a media drive and a removable storage interface. A media drive may include a drive or other mechanism to support fixed or removable storage media, such as a hard disk drive, a floppy disk drive, a magnetic tape drive, an optical disk drive, a CD or DVD drive (R or RW), flash drive, or other removable or fixed media drive. Storage device 124 may further include a computer-readable storage medium having stored therein particular computer software, instructions, or data

Computing device 120 may also include a communications interface 130 that enables software and/or data to be transferred between computing device 120 and one or more external devices, including control inputs 136. Examples of communications interface 130 include a modem, a network interface (such as an Ethernet or other NIC card), a communications port (such as for example, a USB port, a RS-232C serial port), a PCMCIA slot and card, Bluetooth, and the like. Software and data transferred via the communications interface 130 may be in the form of signals, which can be electronic, electromagnetic, optical or other signals capable of being received by communications interface 130. These signals may be transmitted and received by communications interface 130 via a channel, such as a wireless medium, wire or cable, fiber optics, or other communications medium. Control inputs 136 may be communicated to the one or more processors 126 via the communications interface 130. Control inputs 136 may be provided via one or more input devices, such as a keyboard, an interactive display, such as an LCD display configured with touch screen input capabilities, a cursor control, such as a mouse, and so forth. Further, the one or more processors 126 may also be coupled via bus 134 to a display 132, such as a cathode ray tube (CRT) or liquid crystal display (LCD), for displaying information to a user, as well as to an external display 138. For example, one or both of display 132 and external display 138 may be configured to display information from sensors within sequencing chamber 102, thereby enabling a user to enter or set instrument settings and controls via control inputs 136.

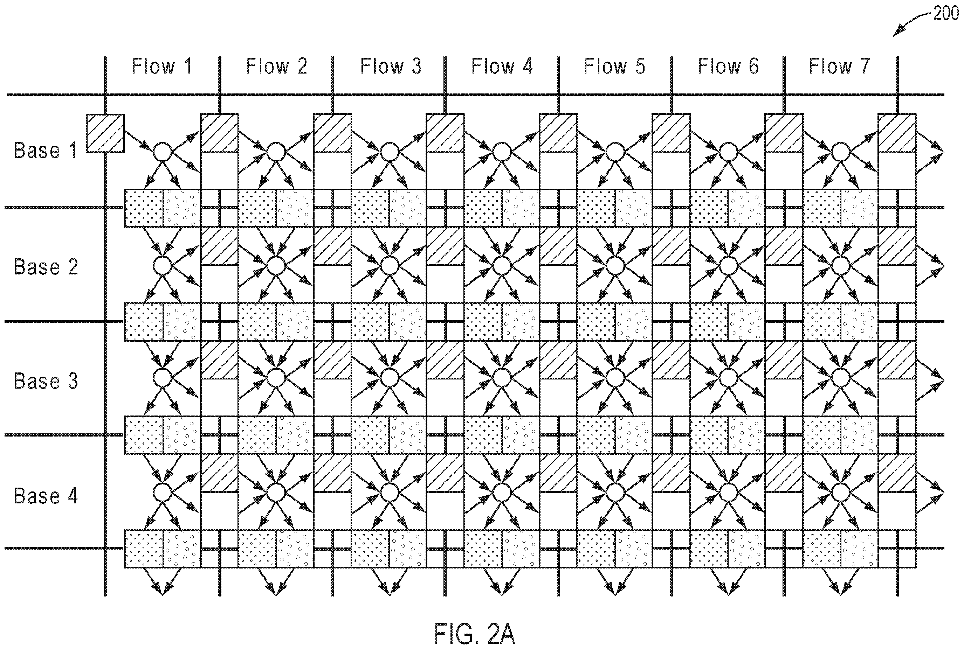

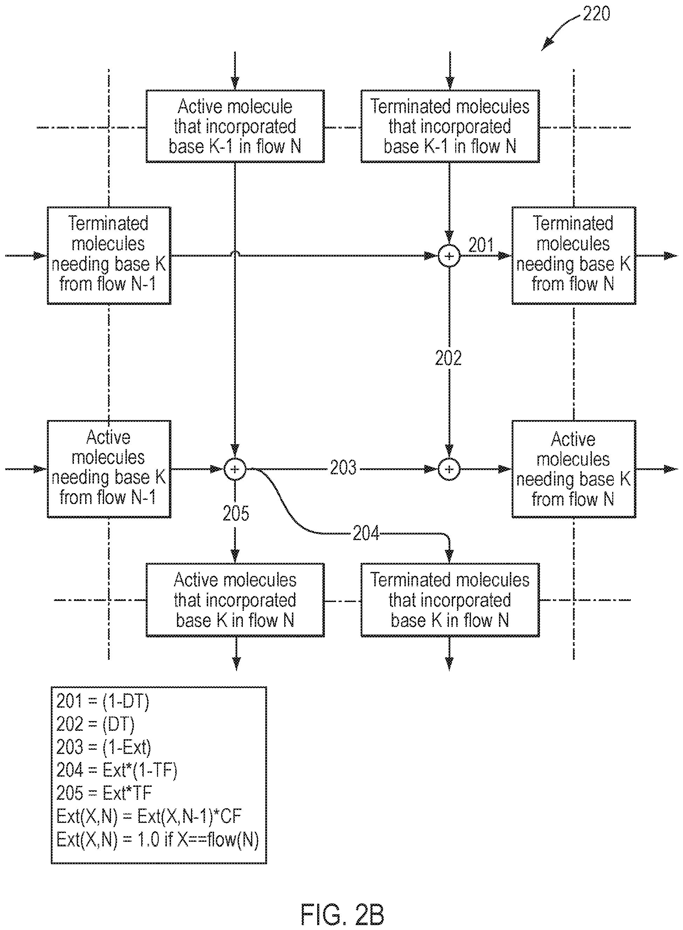

FIGS. 2A-2B illustrate exemplary embodiments of a simulation framework and matrix that can be utilized to calculate predicted sequencing values or measurements for the below-described nucleotide flows (i.e. an ionogram or flowgram). The particular simulation framework and matrix chosen for a given application may depend on a variety of implementation-specific considerations and factors, such as, for example, the type of termination chemistry being utilized in the sequencing-by-synthesis process. For example, FIGS. 2A and 2B illustrate a simulation framework and matrix, respectively, which may be utilized to calculate predicted ionograms in a termination sequencing-by-synthesis process utilizing, for example, reversible or irreversible terminators.

More specifically, FIG. 2A illustrates schematically a simulation framework 200 for calculating predicted ionograms, according to an embodiment of the present disclosure. The representation includes various steps and can be thought of as a matrix of the nucleotide flows (e.g., columns representing flows 1, 2, 3, and so on) and nucleotide bases (e.g., rows representing bases 1, 2, 3, and so on). Bases may or may not incorporate during a particular intended flow, and moreover may incorporate during unintended flows, as described in further detail below. Simulations of intended incorporations, incorporation failures, and/or unintended incorporations generate paths along the cells of such a matrix.

Further, FIG. 2B illustrates an exemplary cell 220 within the matrix 200 illustrated in FIG. 2A, with possible molecule states and state transitions labeled, according to one disclosed embodiment. Such a cell illustrates what may happen for active molecules (*e*.*g*., a molecule being actively synthesized during a flow with an active polymerase) and inactive molecules present at the K-th base during the N-th nucleotide flow. Such a phasing model may be useful in a termination sequencing-by-synthesis platform that uses reversible terminators, for example. To arrive at this point, active molecules include those that either incorporated base K-1 in flow N or that need base K in flow N-1. Terminated molecules include molecules that incorporated base K-1 in flow N, or that need base K from flow N-1.

The terminated molecules that incorporated base K-1 in flow N are summed with the terminated molecules needing base K from flow N-1, upon which there are two possibilities 201 and 202. At 201, a subset of the sum of the terminated molecules remains in the terminated state, therefore proceeding towards needing base K from flow N. At 202, a subset of the sum of the terminated molecules is reactivated and is summed with the results represented by 203 to become the population of active molecules needing base K from flow N. Meanwhile, the active molecules that incorporated base K-1 in flow N are summed with the active molecules that need base K from flow N-1, upon which there are three possibilities 203, 204, and 205. At 203, a subset of the sum of active molecules do not incorporate a base in flow N and join the reactivated molecules 202 to become active molecules needing base K from flow N. At 204, a subset of the sum of active molecules incorporate base K in flow N and terminate, so that they become terminated molecules that incorporated base K in flow N and move to the next cell along a flow column N. Finally, at 205, a subset of the sum of active molecules incorporate base K in flow N and fail to terminate, resulting in the subset of active molecules (i.e. those that did not terminate) that incorporated base K in flow N, and move to the next cell along a flow column N.

Although various embodiments of the present teachings may advantageously be used in connection with pH-based sequence detection, as described herein and in Rothberg et al., U.S. Pat. Appl. Publ. Nos. 2009/0127589 and 2009/0026082 and Rothberg et al., U.K. Pat. Appl. Publ. No. GB2461127, which are all incorporated by reference herein in their entirety, for example, the present teachings may also be used with other detection approaches, including the detection of pyrophosphate (PPi) released by the incorporation reaction (see, e.g., U.S. Pat. Nos. 6,210,891; 6,258,568; and 6,828,100); various fluorescence-based sequencing instrumentation (see, e.g., U.S. Pat. Nos. 7,211,390; 7,244,559; and 7,264,929); some sequencing-by-synthesis techniques that can detect labels associated with the nucleotides, such as mass tags, fluorescent, and/or chemiluminescent labels (in which case an inactivation step may be included in the workflow (e.g., by chemical cleavage or photobleaching) prior to the next cycle of synthesis and detection); and more generally methods where an incorporation reaction generates or results in a product or constituent with a property capable of being monitored and used to detect the incorporation event, including, for example, changes in magnitude (e.g., heat) or concentration (e.g., pyrophosphate and/or hydrogen ions), and signal (e.g., fluorescence, chemiluminescence, light generation), in which cases the amount of the detected product or constituent may be monotonically related to the number of incorporation events, for example. Such other approaches may likewise benefit from the phase correction, signal enhancement, improved accuracy, and/or noise reduction features of the nucleotide flows approaches described herein.

Further, exemplary embodiments disclosed herein provide different patterns or orders of reagent flows that are designed to maximize throughput while minimizing phase errors. For example, with reference to FIG. 1, depending on the type of the selected sequencing-by-synthesis process and the type of termination chemistry employed, the order and mixture of the dNTPs 108 (and/or ddNTPs) may be varied by the flow controller 104. In an exemplary embodiment, a Sanger sequencing process is selected to be run by sequencing chamber 102, whereby four separate sequencing reactions may be run, each including one of the four types of ddNTPs and the other three dNTPs (*e*.*g*., one reaction would include ddATP, but dGTP, dCTP, and dTTP). For further example, if a dye termination sequencing process is selected to be employed by the sequencing chamber 102, the flow controller 104 may regulate a reaction including all four of the ddNTPs (i.e., ddATP, ddCTP, ddGTP, ddTTP), each coupled to a different color fluorescent marker to enable identification, for example, via a fluorescent based imaging system. Various nucleotide flow orders are discussed or contemplated in Hubbell et al., U.S. Pat. 9,428,807, issued August 30, 2016, the contents of which are incorporated by reference herein in their entirety. In one embodiment, the four different kinds of ddNTPs are added sequentially to the reaction chambers, so that each reaction is exposed to the four different ddNTPs, one at a time. In an exemplary embodiment, the four different kinds of ddNTPs are advanced in the following order: ddATP, ddCTP, ddGTP, ddTTP, ddATP, ddCTP, ddGTP, ddTTP, etc., with each exposure followed by a wash step. A two cycle nucleotide flow order can be represented by: ddATP, ddCTP, ddGTP, ddTTP, ddATP, ddCTP, ddGTP, ddTTP, with each exposure being followed by a wash step. In certain embodiments employing termination chemistry utilizing one or more of the terminators discussed above, each nucleotide flow may lead to a single nucleotide incorporation before primer extension is terminated.

Generally, sequencing reaction steps described herein include one or more of the following steps that are performed in any order. An advancing step is performed to introduce one or more dNTPs or ddNTPs (i.e. tagged nucleotides or terminator nucleotides) by one base (i.e. A, T, C, G, etc.). For convenience, a flow of dATP will sometimes be referred to as "a flow of A" or "an A flow," and a sequence of flows may be represented as a sequence of letters, such as "ATGT" indicating "a flow of dATP, followed by a flow of dTTP, followed by a flow of dGTP, followed by a flow of dTTP." In each flow, a polymerase may generally extend the primer by incorporating the flowed dNTP where the next base in the template strand is the complement of the flowed dNTP. The advancing step may incorporate the tagged or terminator nucleotides to a DNA template. A tag or label on each tagged molecule is associated with a response, such as pH or light, that can be measured. The measuring step is performed for measuring a signal from each tagged or labeled molecule. A total signal of all labeled molecules may be obtained for each well, microwell, bead, or other discrete unit within a measuring or sequencing chamber. Optionally, a finishing step may be performed to incorporate additional molecules using the same base. For example, not every molecule of a specific base is advanced during an advance step, which adds noise to the system over repeated cycles. As the noise increases it becomes harder to differentiate between measured signals for different combinations of bases. Thus, the finishing step may be considered a cleaning-up step, and comprises flowing the same molecules as in the previous advance step without any labels, so as to incorporate more molecules associated with the same base, and minimize noise that adds up over time, thus making it difficult to distinguish from true signal data. A reset step is performed to allow all terminated or incorporated molecules to proceed through the system, such that a subsequent advance step may be performed for a different type of combination of bases. The reset step may be performed with a cleave step for removing labels from all labeled molecules.

The exemplary sequencing reaction steps described below with reference to various figures and embodiments may minimize the need to perform finishing steps by virtue of varying the order of bases. For example, the disclosed sequencing reaction steps mitigate the effect of carry forward (CF) or an incomplete extension (IE). Each exemplary embodiment described below comprises slightly different sequencing reaction steps, such as different nucleotide flow orders, and may be considered as performing a different number of advance steps per cleave step so as to explore the trade-offs for corresponding amounts of phase error protection or minimization. For example, repeatedly performing advance steps for each of four different nucleotides (A, G, T, C) without variance may reduce phase error protection. As the number of advance steps is reduced, and variations in base order introduced, more phase error protection is ensured. Thus, varying the sequencing reaction steps and nucleotide flow orders ensures that phase error build-up, CF, or IE for a specific nucleotide are minimized. Further, reducing the number of rinse/wash/cleave steps improves throughput of these methods.

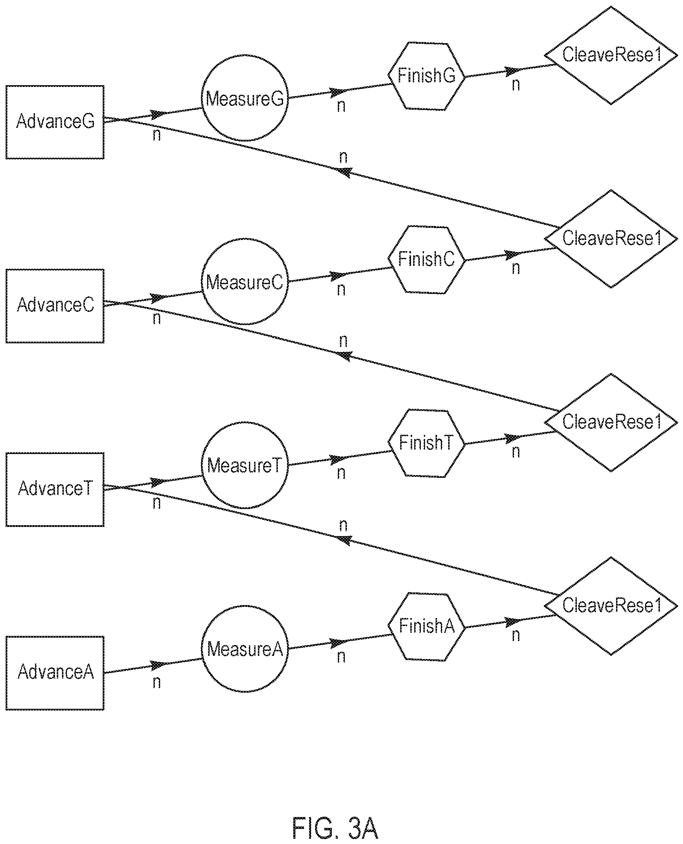

FIG. 3A illustrates sequencing reaction steps comprising advance, measure, finish, and cleave/reset steps for a single nucleotide per sequence, according to an exemplary embodiment of the present disclosure. According to this embodiment, each dNTP is individually advanced (depicted by a square in the figure), measured (depicted by a circle in the figure), finished (depicted by a hexagon in the figure), and reset/cleaved (depicted by a diamond in the figure). The exemplary sequencing reaction steps may be represented as "A, T, C, G" with a measurement, finish, and cleave/reset step per nucleotide. Although not shown herein, a wash step is optionally added at any point in the cycle; for example, subsequent to each cleave/reset step.

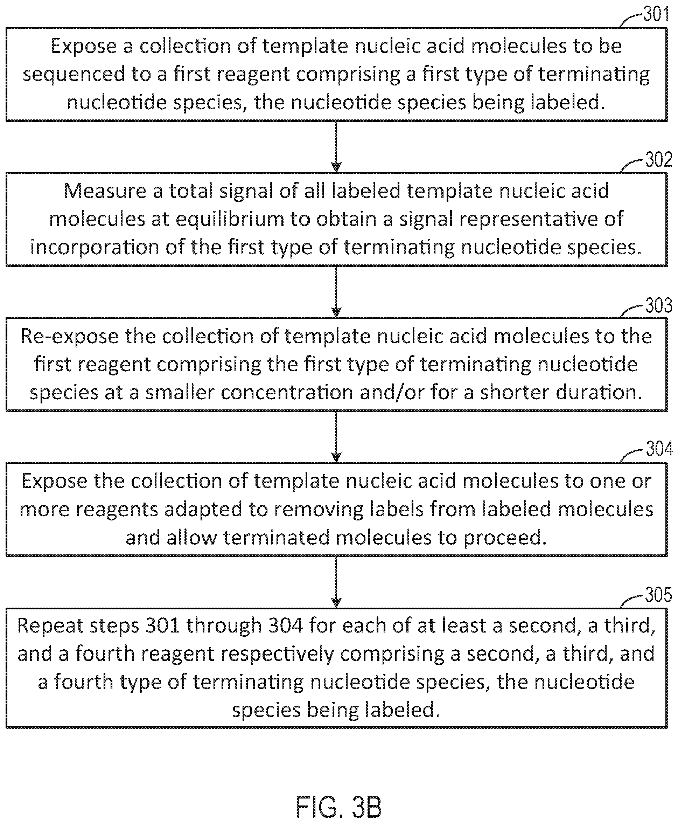

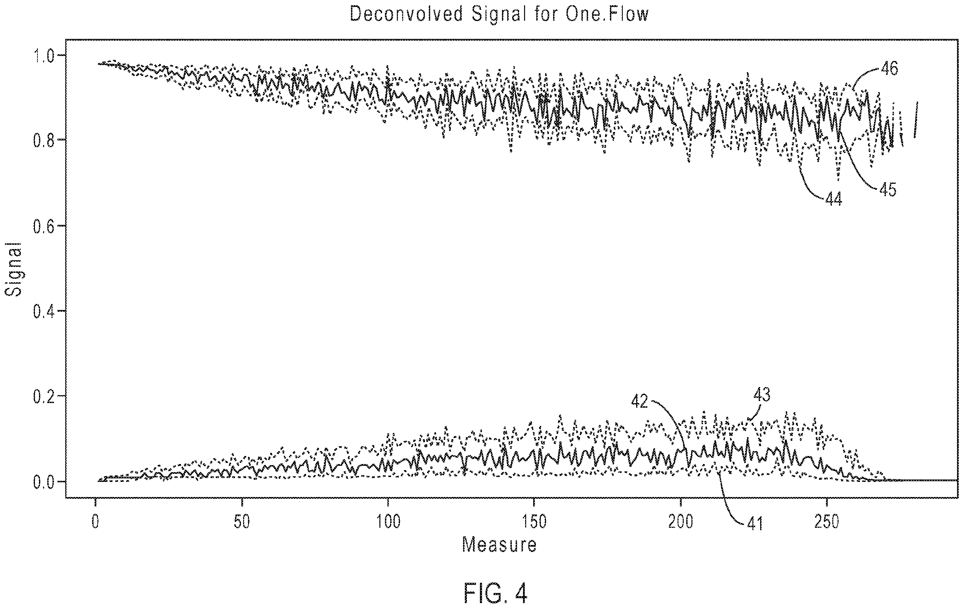

FIG. 3B is a flow chart illustrating a method for performing a nucleotide flow based on the sequencing reaction steps of FIG. 3A. At 301, an advance step exposes a collection of template nucleic acid molecules intended to be sequenced to a first reagent comprising a first type of nucleotide or terminating nucleotide species. The first reagent may be labeled with a labeling reagent that is associated with a response, such as pH or light, that can be measured. At 302, a total signal of all labeled molecules is measured at equilibrium, to obtain a signal representative of incorporation of the first type of nucleotide or terminating nucleotide species. Signal response curves corresponding to this step are further illustrated below in FIG. 4. Subsequently, at 303, a finish step is performed to re-expose the template molecules to the first reagent at a smaller concentration and/or for a shorter duration. At 304, cleave and reset steps are performed to expose the template(s) to cleaving agents to remove labels from the labeled molecules, and to allow the terminated molecules to proceed through the system. Subsequently, as illustrated at step 305, steps 301-304 are repeated for each of at least a second, a third, and a fourth reagent respectively comprising a second, a third, and a fourth type of nucleotide / terminating nucleotide species that are correspondingly labeled.

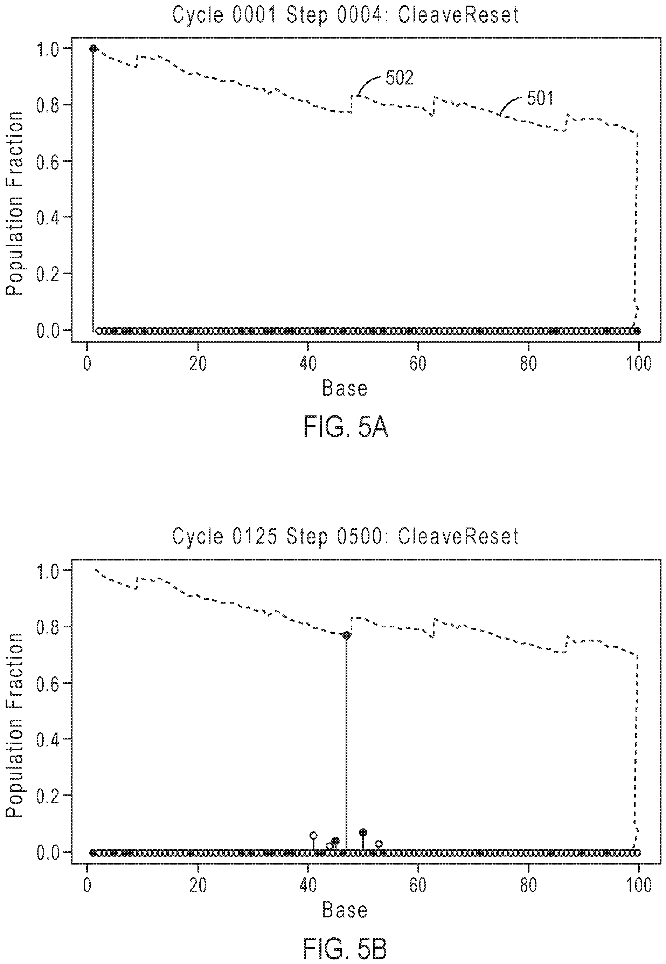

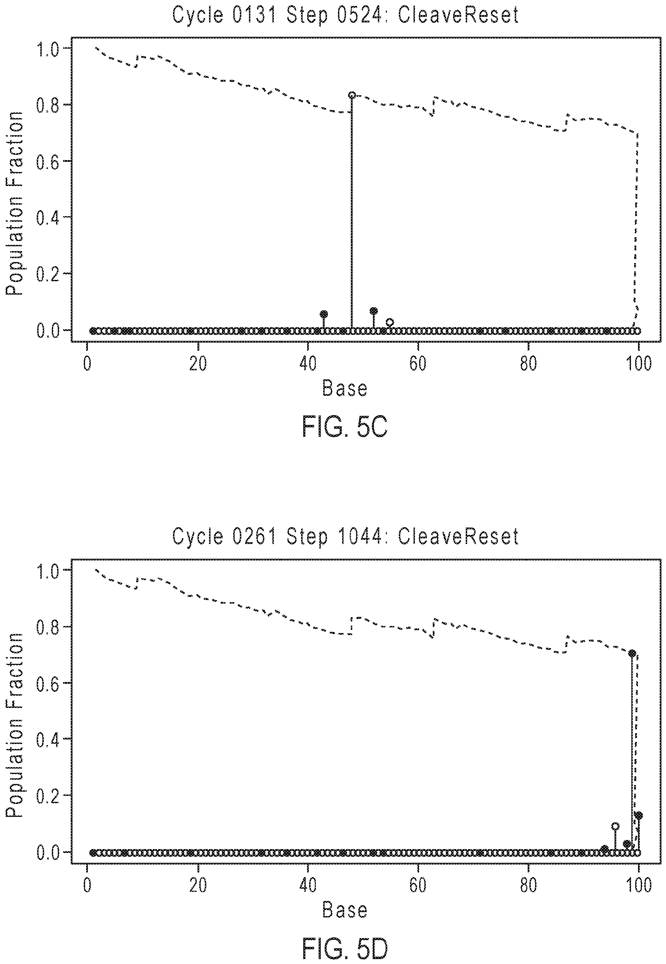

FIG. 4 illustrates exemplary simulation data corresponding to signal response curves for the sequencing reaction steps of FIG. 3A. The exemplary simulation data illustrated herein (and in subsequent depictions of simulation data illustrated hereafter, for instance in FIGS. 5A-5D, 6A-6D, 8, 9A-9D, etc.) are based on the exemplary simulation framework illustrated in FIGS. 2A-2B. With reference to FIG. 4, signal response curves are depicted with signal intensity on the y-axis and the n^{th} flow number (time) on the x-axis, with two triplet sets of plot lines illustrated, each of the triplet sets having a darker solid line (42, 45) in the middle between two lighter dotted lines (41, 43; 44, 46). The bottom triplet set of plot lines (41, 42, 43) show the signal from 0-mer events (non-incorporation); and the top triplet set of plot lines (44, 45, 46) show the signal from 1-mer or 2-mer incorporation events. Within each triplet set, the darker solid line in the middle (42, 45) represents the median signal, the lighter dotted line above (43, 46) represents the 25 percentile signal, and the lighter dotted line below (41, 44) represents the 75 percentile signal. As shown in FIG. 4, while the signal for the 1-mer/2-mer incorporation events degrades as the sequencing read progresses, the signal produced by non-incorporation 0-mer events (e.g., the background signal) increases as the sequencing read progresses. Thus, at later portions of the sequencing read, the signal resolution diminishes and it becomes more difficult to distinguish the 0-mer events from 1-mer/2-mer events. As explained above, the accumulated effects of CF and IE events contribute to this degradation of signal quality.

FIGS. 5A-5D illustrate exemplary simulation data corresponding to template population evolution as sequencing progresses for the sequencing reaction steps of FIG. 3A. The y-axis represents the population fraction, with the plot line representing the population indicating that the relative number of in-sync templates decreases over time with progression of the sequencing read due to the loss of phase synchrony. Dashed line 501 corresponds to an in-phase population that generally decreases over time, with phase corrections depicted by zig-zag jumps such as 502, which are caused by phase correcting flow orders that allow for out of phase populations to rejoin the population.

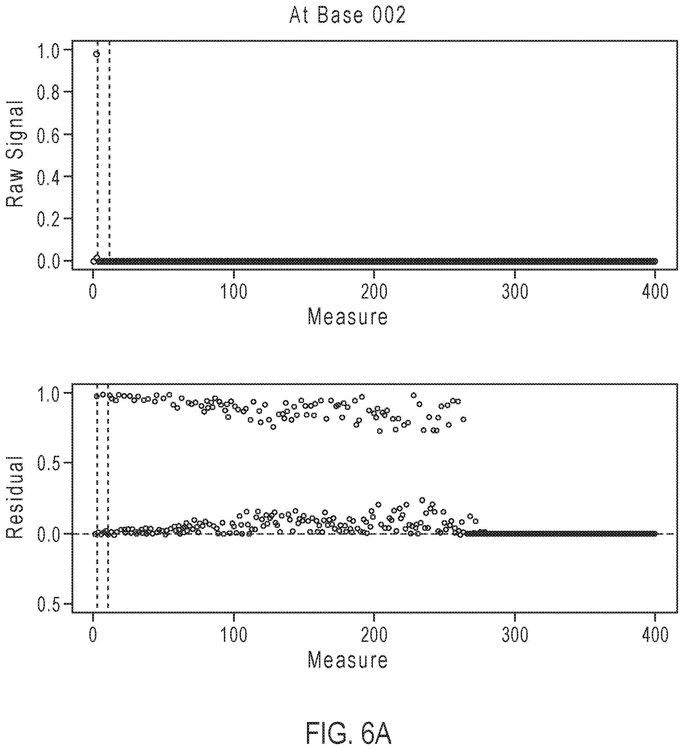

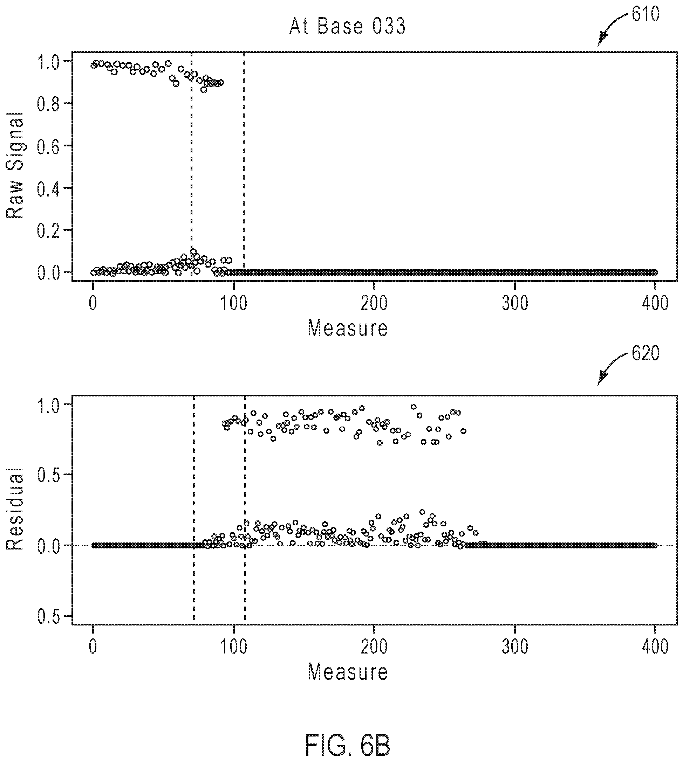

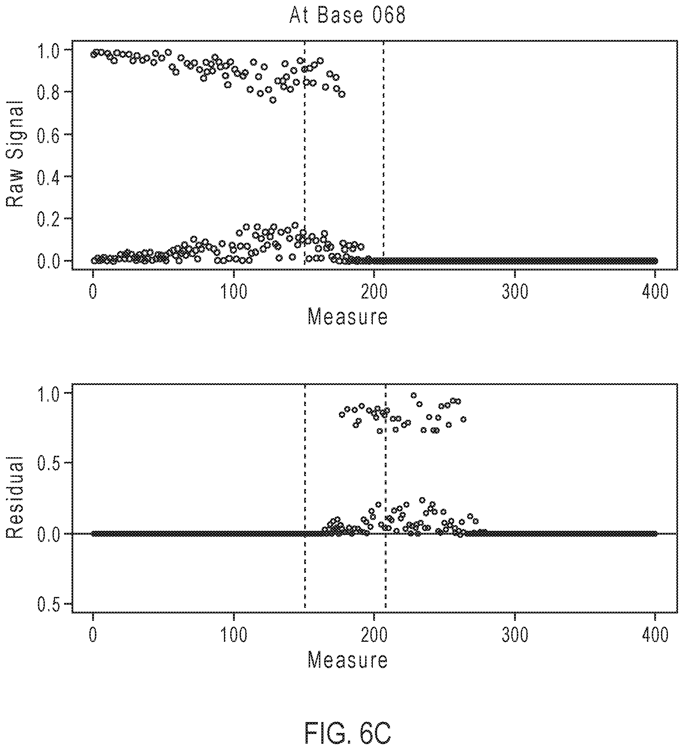

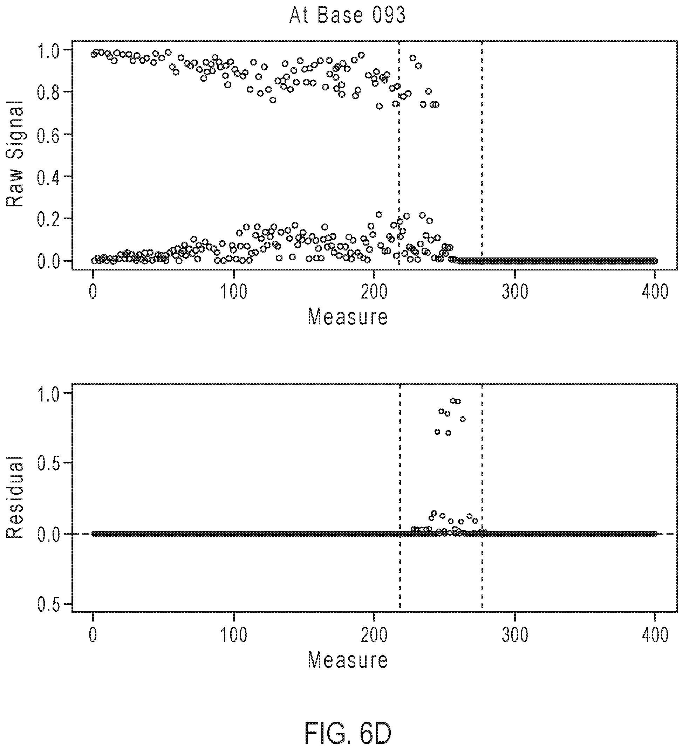

FIGS. 6A-6D illustrate exemplary simulation data corresponding to partially base-called simulated sequences for the sequencing reaction steps of FIG. 3A. The top row of each of FIGS 6A-6D depicted by reference numeral 610 shows the predicted signal as obtained through the called sequence and the simulation framework, whereas the bottom row 620 shows a simulated measured signal that is not yet base-called.

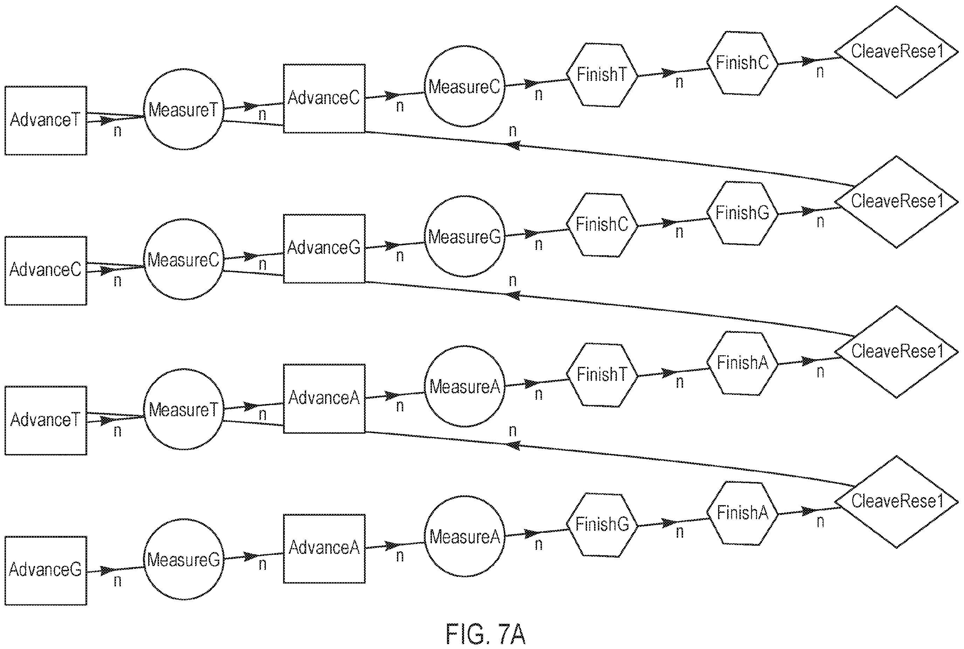

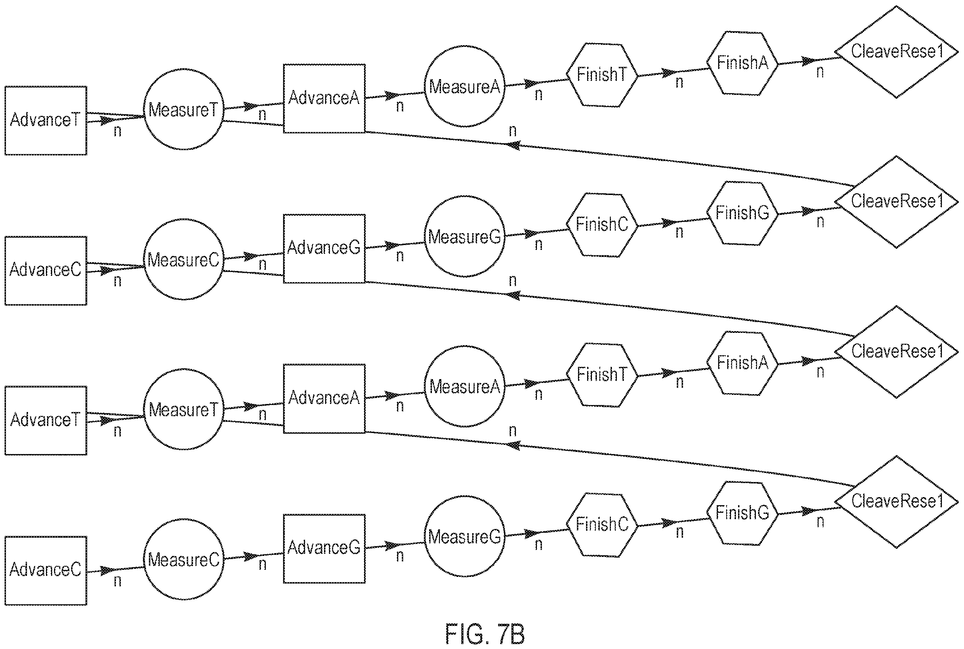

FIGS. 7A-7B are variations of sequencing reaction steps comprising advance and measure steps for two different terminating nucleotides for every finish and cleave/reset step per sequence, according to another exemplary embodiment of the present disclosure. According to this embodiment, two different dNTPs are individually advanced and measured prior to both being finished and reset/cleaved. The exemplary nucleotide flow order in FIG. 7A may be represented as "GA, CA, CG, TC", with a measurement step in between each advance, and finish and cleave/reset steps in between each pair of nucleotides. In contrast, the exemplary nucleotide flow order in FIG. 7B may be represented as "CG, TA, CG, TA", which comprises fewer combinations of pairs than the nucleotide flow order illustrated in FIG. 7A. Although not shown herein, a wash step is optionally added at any point in the cycle; for example, subsequent to each cleave/reset step.

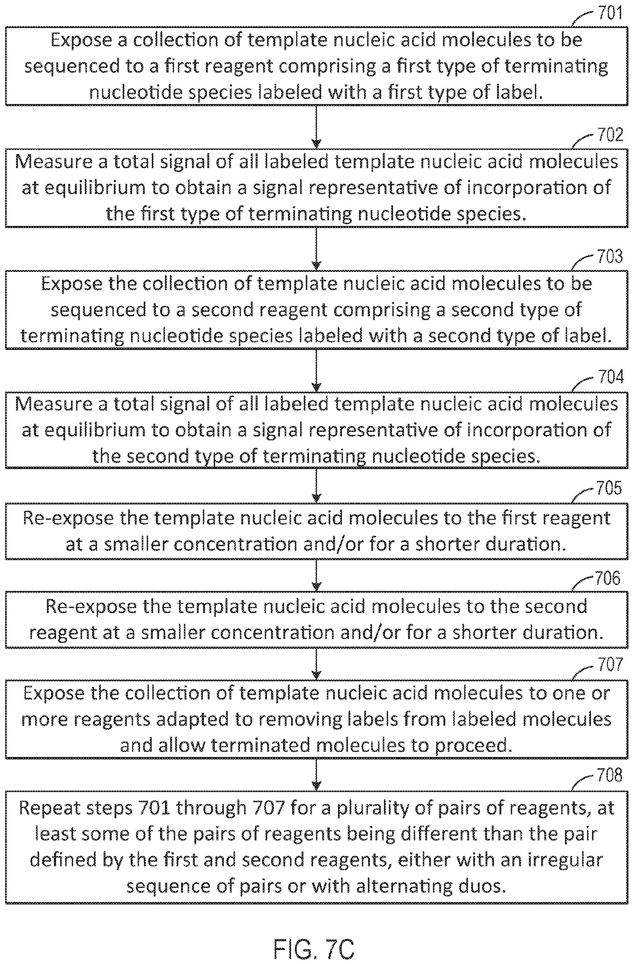

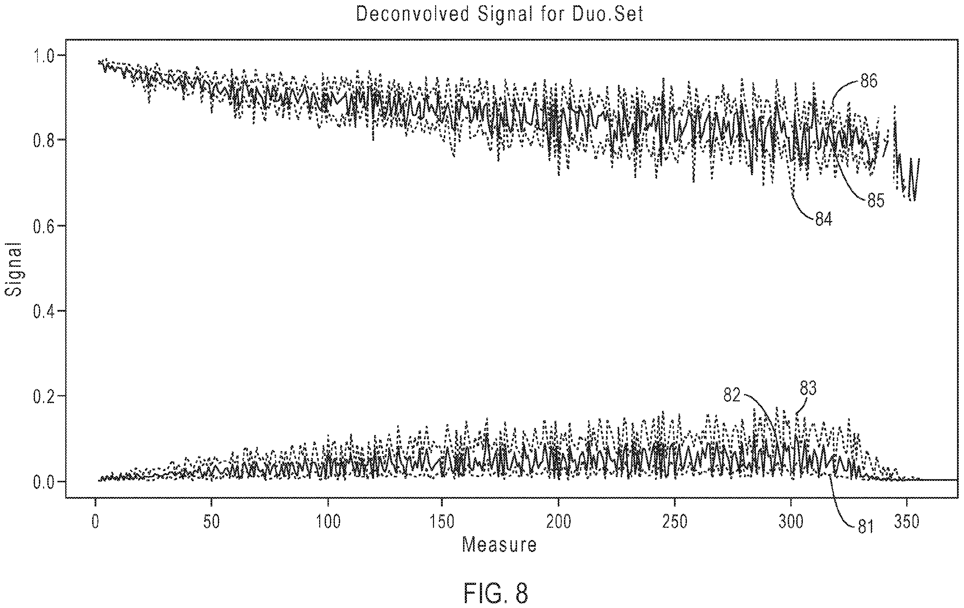

FIG. 7C is a flow chart illustrating a method for performing a nucleotide flow based on the sequencing reaction steps of FIGS. 7A-7B, according to an embodiment of the present disclosure. At 701, an advance step exposes a collection of template nucleic acid molecules intended to be sequenced to a first reagent comprising a first type of nucleotide or terminating nucleotide species. The first reagent may be labeled with a labeling reagent that is associated with a response, such as pH or light, that can be measured. At 702, a total signal of all labeled molecules is measured at equilibrium, to obtain a signal representative of incorporation of the first type of nucleotide or terminating nucleotide species. Subsequently at 703, another advance step exposes a collection of template nucleic acid molecules intended to be sequenced to a second reagent comprising a second type of nucleotide or terminating nucleotide species. The second reagent may be labeled with a labeling reagent that is associated with a response different from the first reagent, such as pH or light. At 704, a total signal of all labeled molecules is measured at equilibrium, to obtain a signal representative of incorporation of the second type of nucleotide or terminating nucleotide species. Signal response curves corresponding to steps 702 and 704 are further illustrated below in FIG. 8. Subsequently, at 705 and 706, finish steps are performed to re-expose the template molecules respectively to the first and second reagents at a smaller concentration and/or for a shorter duration. At 707, cleave and reset steps are performed to expose the template(s) to cleaving agents to remove labels from the labeled molecules, and to allow the terminated molecules to proceed through the system. Finally, at step 708, steps 701-707 are repeated for each of a plurality of pairs of reagents respectively comprising a pair of nucleotide / terminating nucleotide species that are correspondingly labeled and that are different from the pair comprising the first and second nucleotides.

FIG. 8 illustrates exemplary simulation data corresponding to signal response curves for the sequencing reaction steps of FIG. 7A, according to an embodiment of the present disclosure. Signal response curves are depicted with signal intensity on the y-axis and the nth flow number (time) on the x-axis, with two triplet sets of plot lines illustrated, each of the triplet sets having a darker solid line (82, 85) in the middle between two lighter dotted lines (81, 83; 84, 86). The bottom triplet set of plot lines (81, 82, 83) show the signal from 0-mer events (non-incorporation); and the top triplet set of plot lines (84, 85, 86) show the signal from 1 -mer or 2-mer incorporation events. Within each triplet set, the darker solid line in the middle (82, 85) represents the median signal, the lighter dotted line above (83, 86) represents the 25 percentile signal, and the lighter dotted line below (81, 84) represents the 75 percentile signal. As shown in FIG. 8, while the signal for the 1-mer/2-mer incorporation events degrades as the sequencing read progresses, the signal produced by non-incorporation 0-mer events (e.g., the background signal) increases as the sequencing read progresses. Thus, at later portions of the sequencing read, the signal resolution diminishes and it becomes more difficult to distinguish the 0-mer events from 1-mer/2-mer events. As explained above, the accumulated effects of CF and/or IE events contribute to this degradation of signal quality.

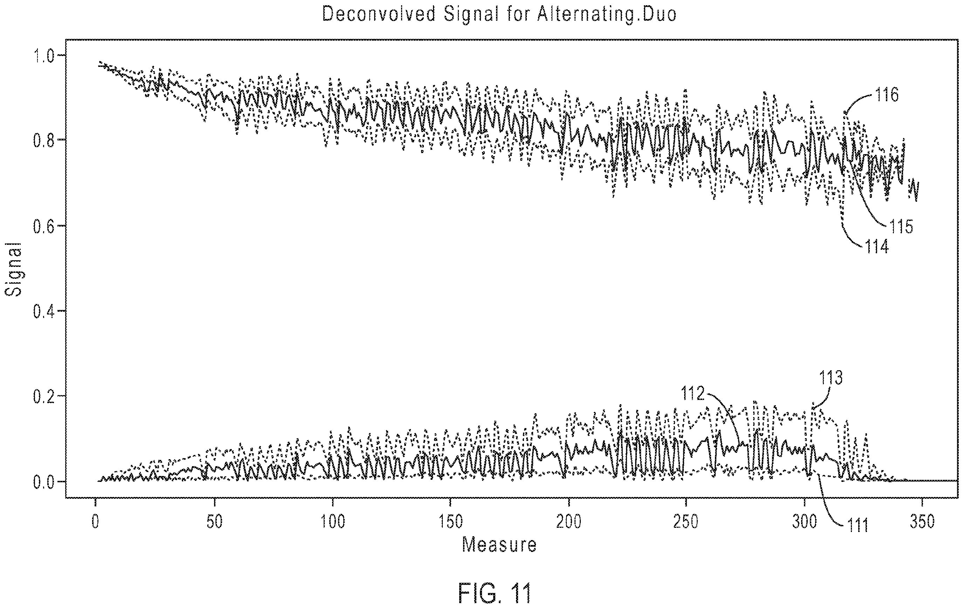

Similarly, FIG. 11 illustrates exemplary simulation data corresponding to signal response curves for the sequencing reaction steps of FIG. 7B, according to an embodiment of the present disclosure. As is evident in a comparison between FIG. 11 and FIG. 8, signal resolution for the sequencing reaction steps of FIG. 7B diminishes to a greater degree relative to the signal resolution for the sequencing reaction steps of FIG. 7A. This may be attributed to the increased variability of pair combinations in the nucleotide flow order of FIG. 7A.

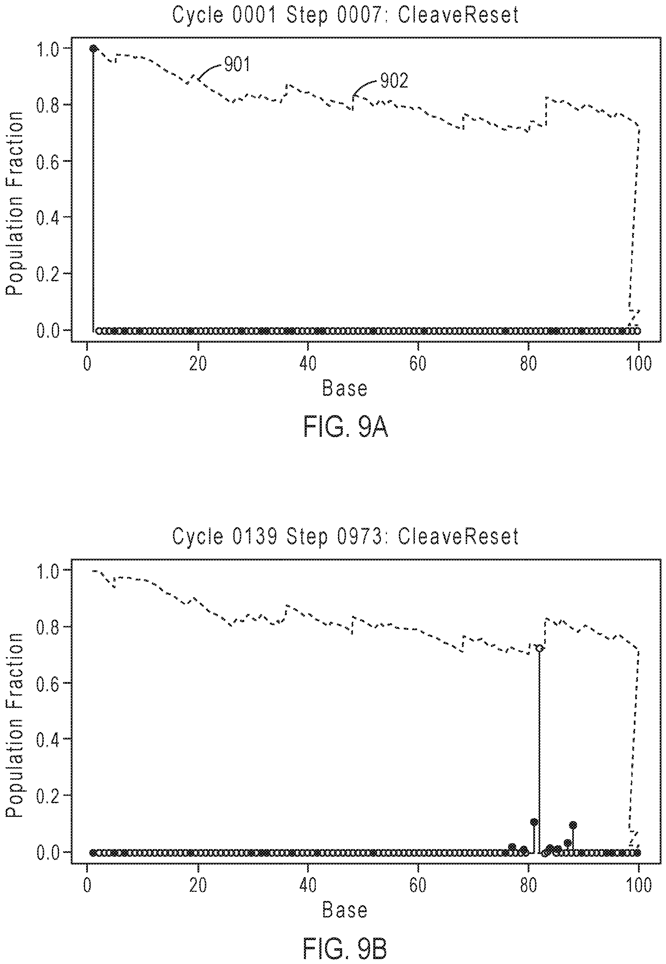

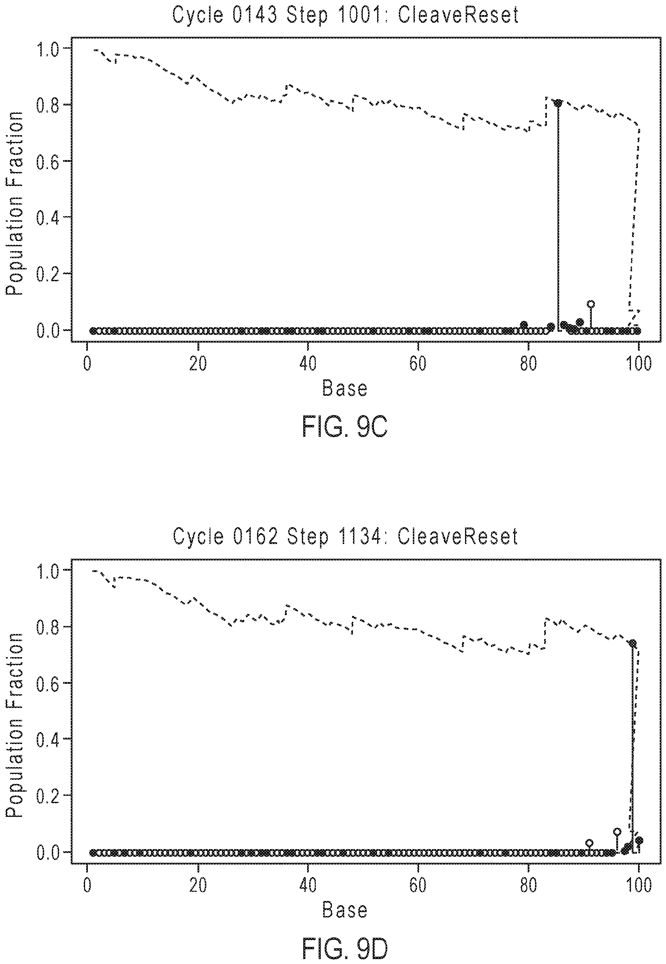

FIGS. 9A-9D illustrate exemplary simulation data corresponding to template population evolution as sequencing progresses for the sequencing reaction steps of FIG. 7A, according to an embodiment of the present disclosure. The y-axis represents the population fraction, with the plot line representing the population indicating that the relative number of in-sync templates decreases over time with progression of the sequencing read due to the loss of phase synchrony. Dashed line 901 corresponds to an in-phase population that generally decreases over time, with phase corrections depicted by zig-zag jumps such as 902, which are caused by phase correcting flow orders that allow for out of phase populations to rejoin the population. As is evident in FIGS. 9A-9D, flow orders that allow for out of phase populations to rejoin may have increases in the ideal in-phase population in specific points in time.

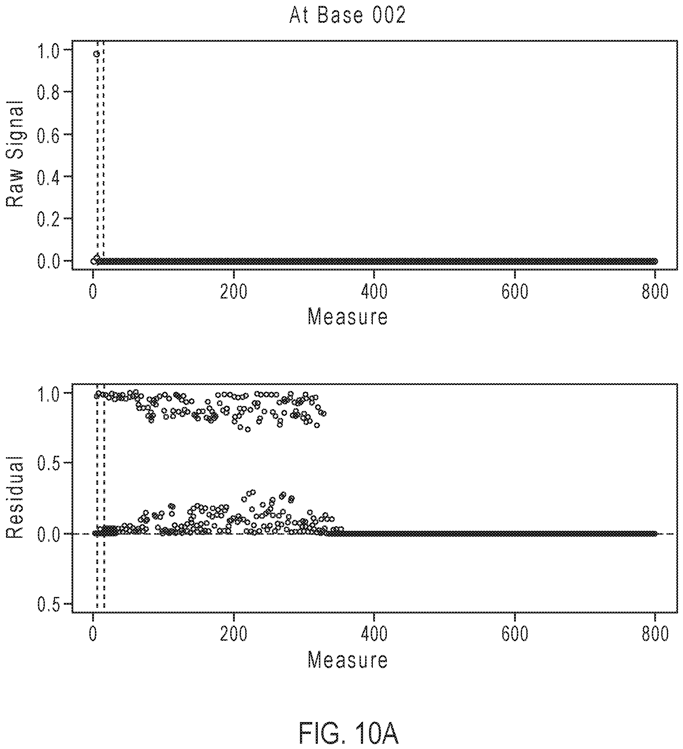

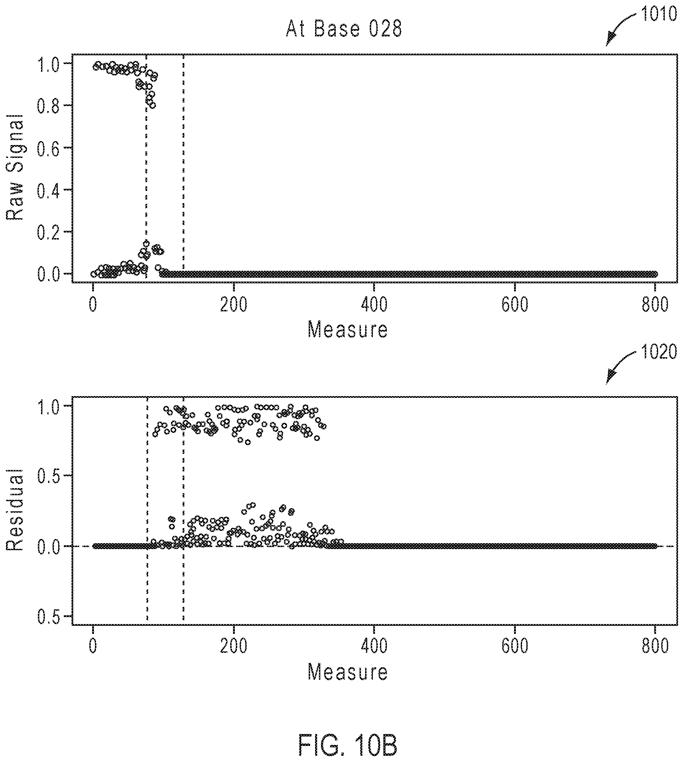

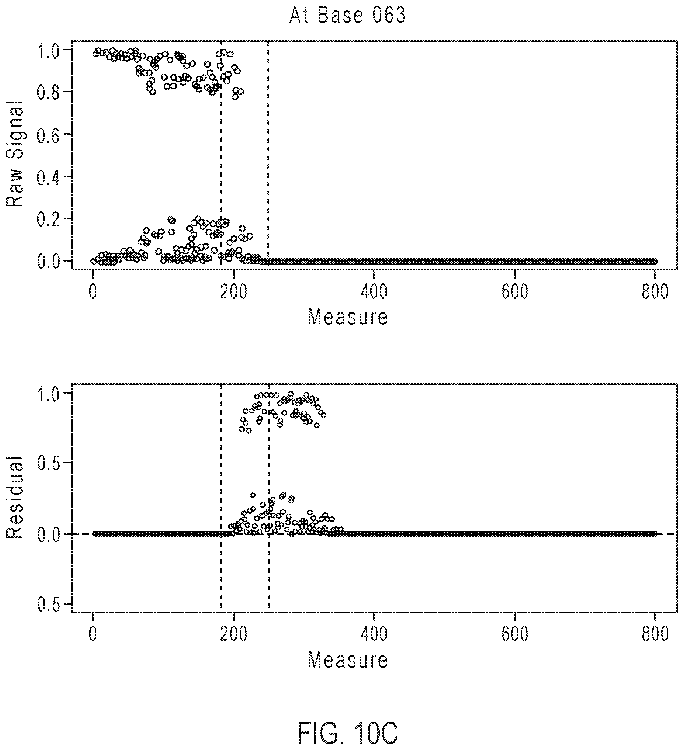

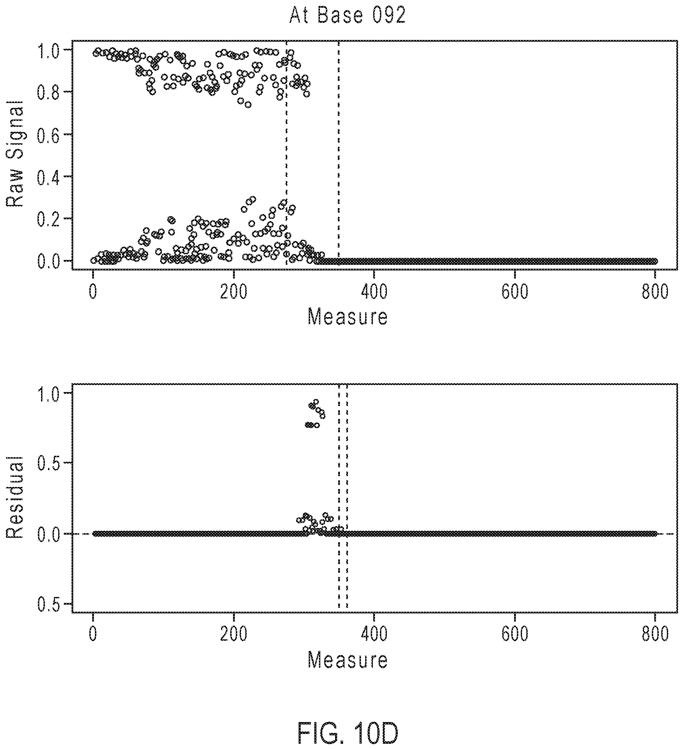

FIGS. 10A-10D illustrate exemplary simulation data corresponding to partially base-called simulated sequences for the sequencing reaction steps of FIG. 7A. The top row of each of FIGS 10A-10D depicted by reference numeral 1010 shows the predicted signal as obtained through the called sequence and the simulation framework, whereas the bottom row 1020 shows a simulated measured signal that is not yet base-called.

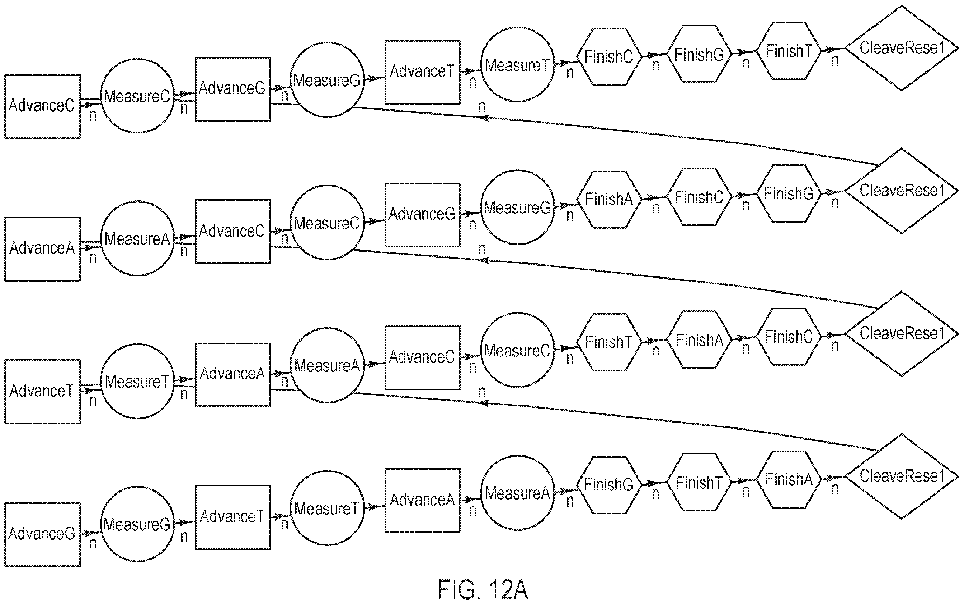

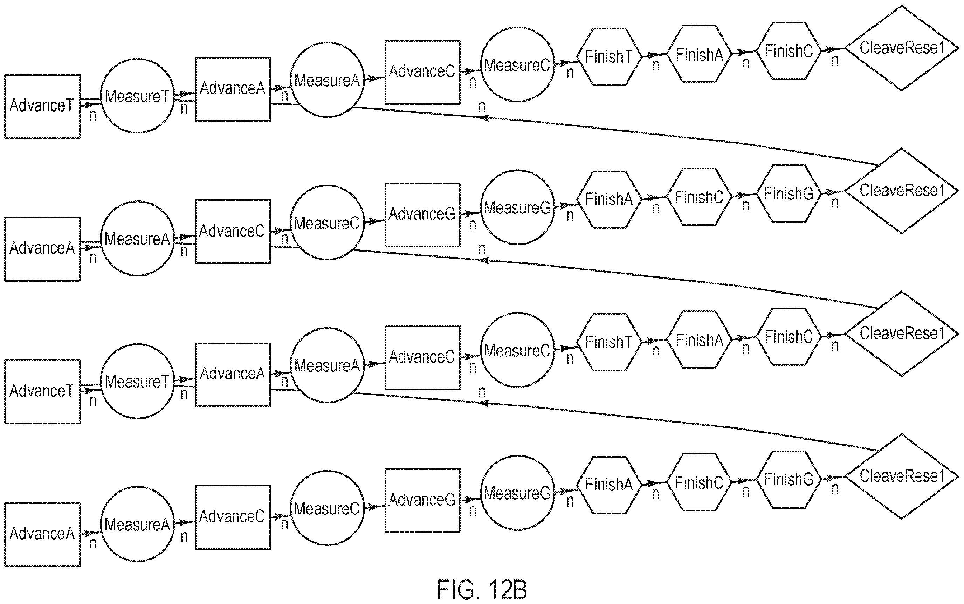

FIGS. 12A-12B are variations of sequencing reaction steps comprising advance and measure steps for three different terminating nucleotides for every finish and cleave/reset step per sequence, according to an exemplary embodiment of the present disclosure. According to this embodiment, three dNTPs (i.e. a "triplet") are individually advanced and measured prior to the triplet being finished and reset/cleaved. The exemplary nucleotide flow order in FIG. 12A may be represented as "GTA, TAC, ACG, CGT" with a measurement step in between each advance, and finish and cleave/reset steps in between each pair of nucleotides. In contrast, the exemplary nucleotide flow order in FIG. 12B may be represented as "ACG, TAC, ACG, TAC", which comprises fewer combinations of triplets than the nucleotide flow order illustrated in FIG. 12A. Although not shown herein, a wash step is optionally added at any point in the cycle; for example, subsequent to each cleave/reset step.

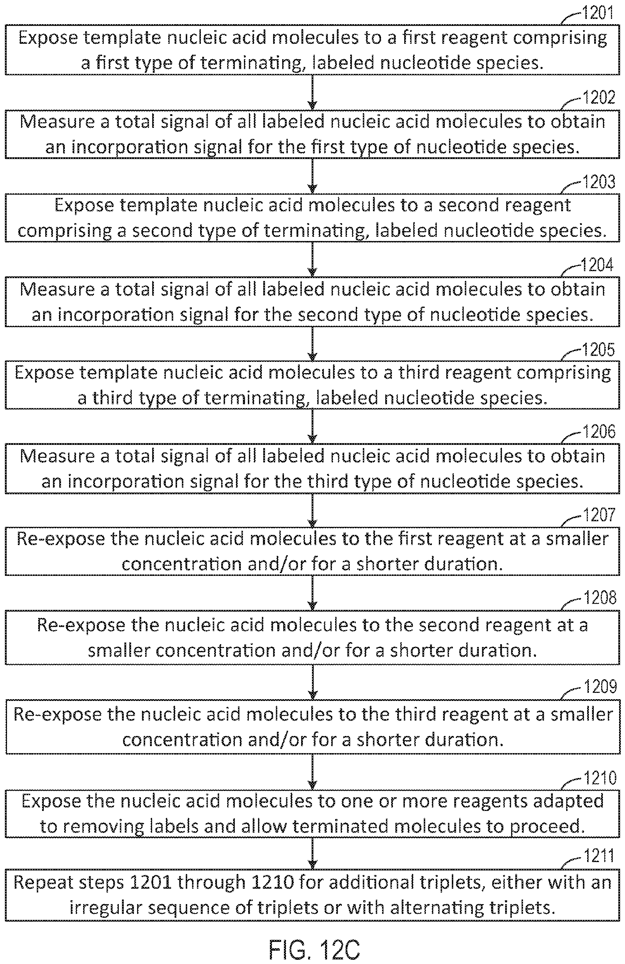

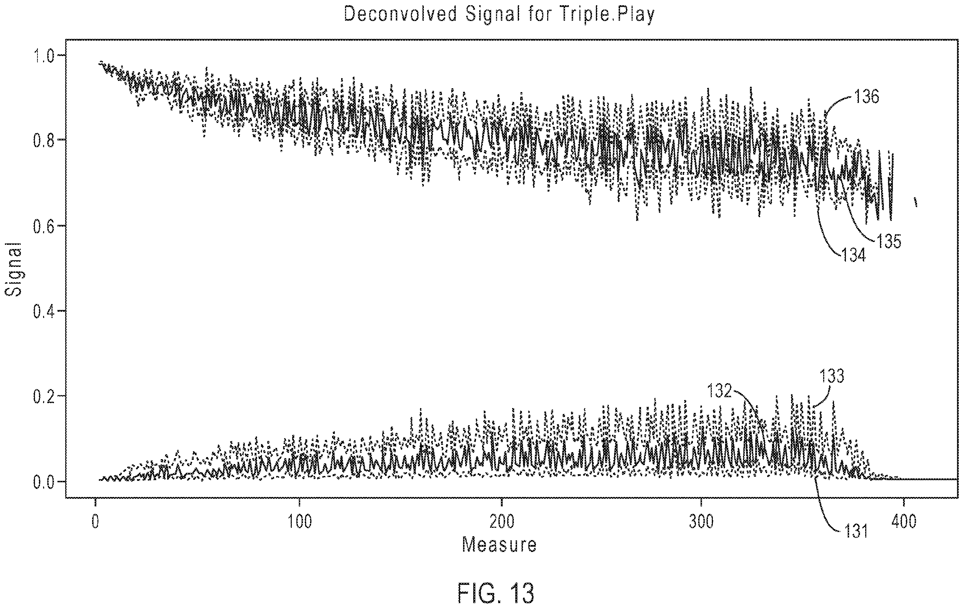

FIG. 12C is a flow chart illustrating a method for performing a nucleotide flow based on the sequencing reaction steps of FIGS. 12A-12B, according to an embodiment of the present disclosure. At 1201, an advance step exposes a collection of template nucleic acid molecules intended to be sequenced to a first reagent comprising a first type of nucleotide or terminating nucleotide species. The first reagent may be labeled with a labeling reagent that is associated with a response, such as pH or light, that can be measured. At 1202, a total signal of all labeled nucleotides is measured at equilibrium, to obtain a signal representative of incorporation of the first type of nucleotide or terminating nucleotide species. Subsequently at 1203, another advance step exposes a collection of template nucleic acid molecules intended to be sequenced to a second reagent comprising a second type of nucleotide or terminating nucleotide species. The second reagent may be labeled with a labeling reagent that is associated with a response different from the first reagent, such as pH or light. At 1204, a total signal of all labeled nucleotides is measured at equilibrium, to obtain a signal representative of incorporation of the second type of nucleotide or terminating nucleotide species. Further, at 1205, another advance step exposes a collection of template nucleic acid molecules intended to be sequenced to a third reagent comprising a third type of nucleotide or terminating nucleotide species. The third reagent may be labeled with a labeling reagent that is associated with a response different from the first and second reagents, such as pH or light. At 1206, a total signal of all labeled nucleotides is measured at equilibrium, to obtain a signal representative of incorporation of the third type of nucleotide or terminating nucleotide species. Signal response curves corresponding to steps 1202, 1204, and 1206 are further illustrated below in FIG. 13.

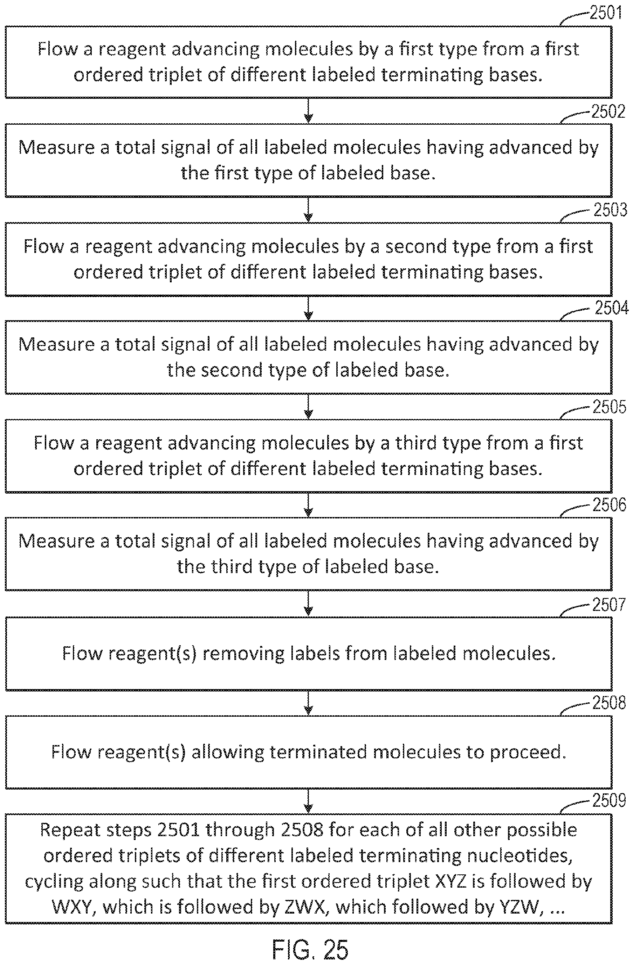

Subsequently, at 1207-1209, finish steps are performed to re-expose the template molecules respectively to the first, second, and third reagents at a smaller concentration and/or for a shorter duration. At 1210, cleave and reset steps are performed to expose the template(s) to cleaving agents to remove labels from the labeled molecules, and to allow the terminated molecules to proceed through the system. Finally, steps 1201-1210 are repeated for each of a plurality of reagents respectively comprising a triplet of nucleotide / terminating nucleotide species that are correspondingly labeled and that are different from the triplet comprising the first, second, and third nucleotides. Further, FIG. 25 below illustrates a flowchart similar to that of FIG. 12C, with the exception of the finish steps, and repeating the sequence using cyclic ordering of triplets.

FIG. 13 illustrates exemplary simulation data corresponding to signal response curves for the sequencing reaction steps of FIG. 12A. Signal response curves are depicted with signal intensity on the y-axis and the nth flow number (time) on the x-axis, with two triplet sets of plot lines illustrated, each of the triplet sets having a darker solid line (132, 135) in the middle between two lighter dotted lines (131, 133; 134, 136). The bottom triplet set of plot lines (131, 132, 133) show the signal from 0-mer events (non-incorporation); and the top triplet set of plot lines (134, 135, 136) show the signal from 1-mer or 2-mer incorporation events. Within each triplet set, the darker solid line in the middle (132, 135) represents the median signal, the lighter dotted line above (133, 136) represents the 25 percentile signal, and the lighter dotted line below (131, 134) represents the 75 percentile signal. As shown in FIG. 13, while the signal for the 1-mer/2-mer incorporation events degrades as the sequencing read progresses, the signal produced by non-incorporation 0-mer events (e.g., the background signal) increases as the sequencing read progresses. Thus, at later portions of the sequencing read, the signal resolution diminishes and it becomes more difficult to distinguish the 0-mer events from 1-mer/2-mer events. As explained above, the accumulated effects of CF and/or IE events contribute to this degradation of signal quality.

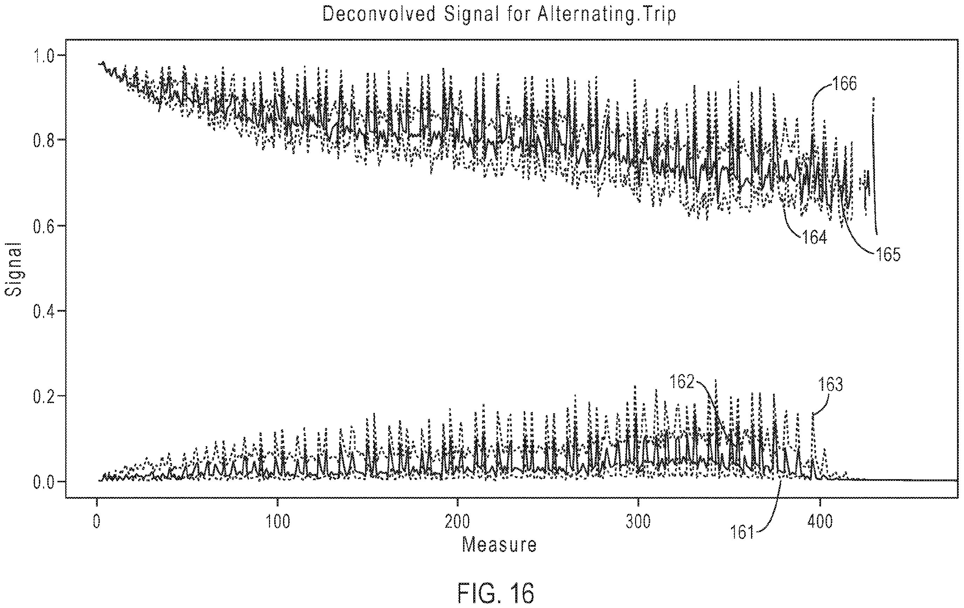

Similarly, FIG. 16 illustrates exemplary simulation data corresponding to signal response curves for the sequencing reaction steps of FIG. 12B, according to an embodiment of the present disclosure. As is evident in a comparison between FIG. 16 and FIG. 13, signal resolution for the sequencing reaction steps of FIG. 12B diminishes to a greater degree relative to the signal resolution for the sequencing reaction steps of FIG. 12A. This may be attributed to the increased variability of triplet combinations in the nucleotide flow order of FIG. 12A.

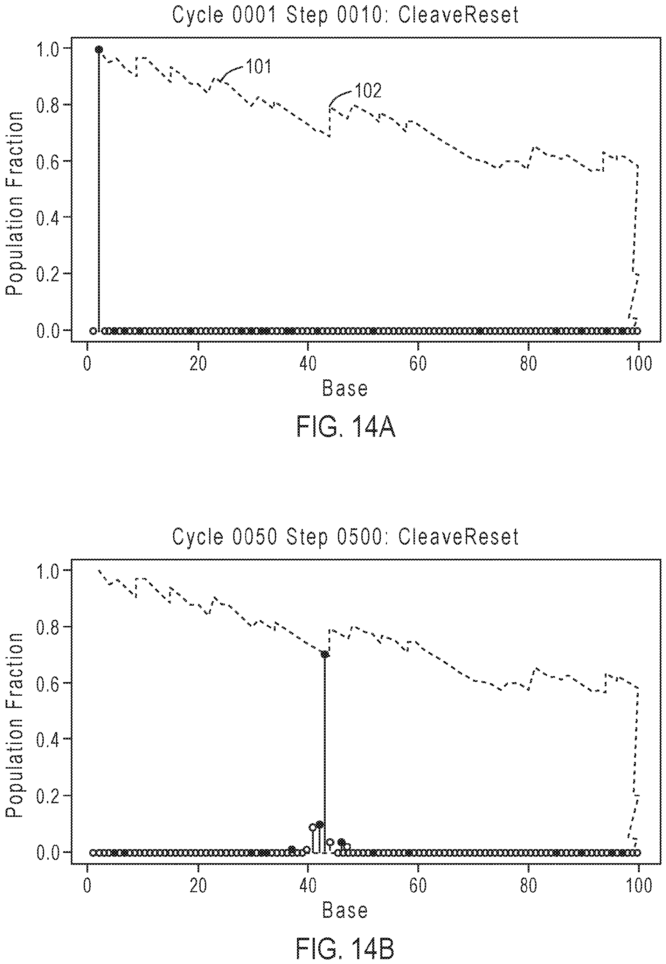

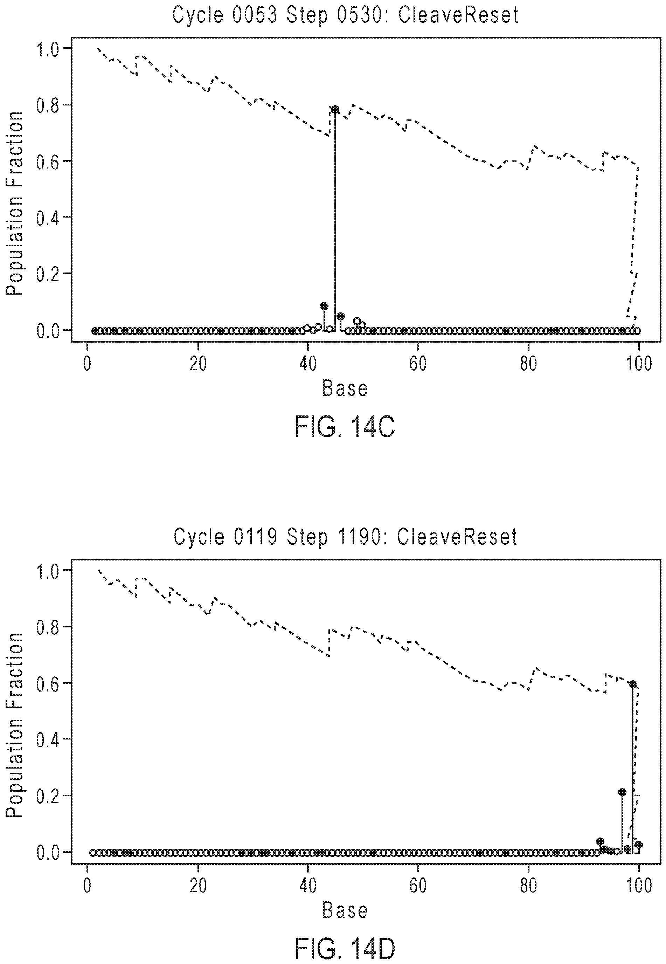

FIGS. 14A-14D illustrate exemplary simulation data corresponding to template population evolution as sequencing progresses for the sequencing reaction steps of FIG. 12A, according to an embodiment of the present disclosure. The y-axis represents the population fraction, with the plot line representing the population indicating that the relative number of in-sync templates decreases over time with progression of the sequencing read due to the loss of phase synchrony. Dashed line 1401 corresponds to an in-phase population that generally decreases over time, with phase corrections depicted by zig-zag jumps such as 1402, which are caused by phase correcting flow orders that allow for out of phase populations to rejoin the population. As is evident in FIGS. 14A-14D, flow orders that allow for out of phase populations to rejoin may have increases in the ideal in-phase population in specific points in time.

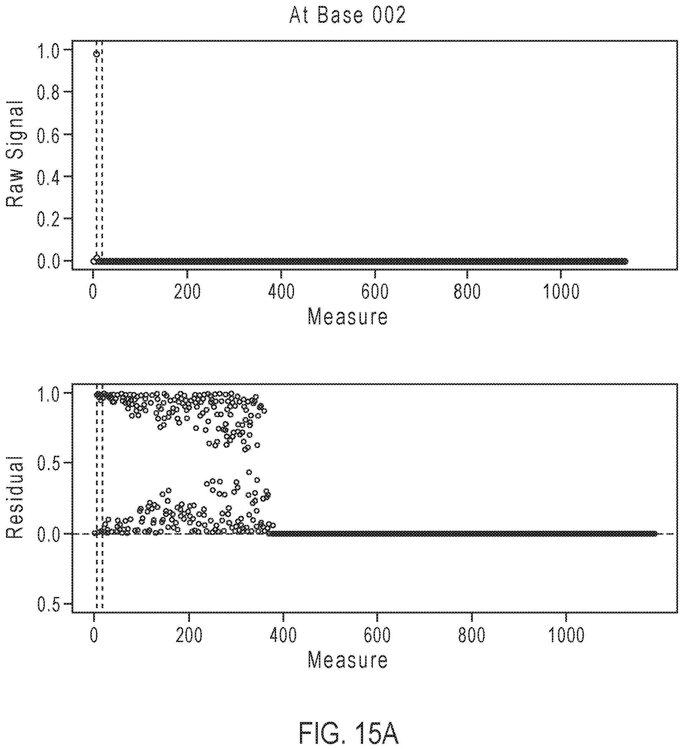

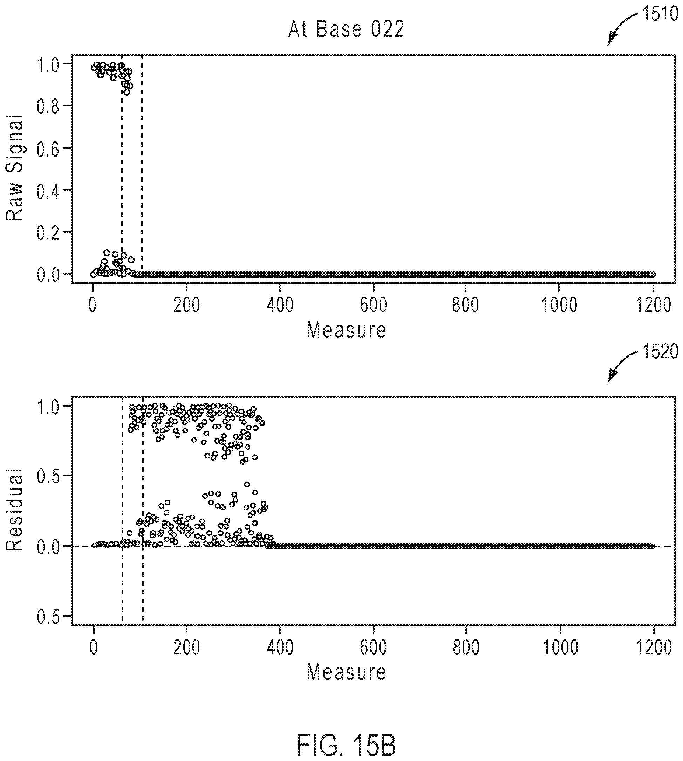

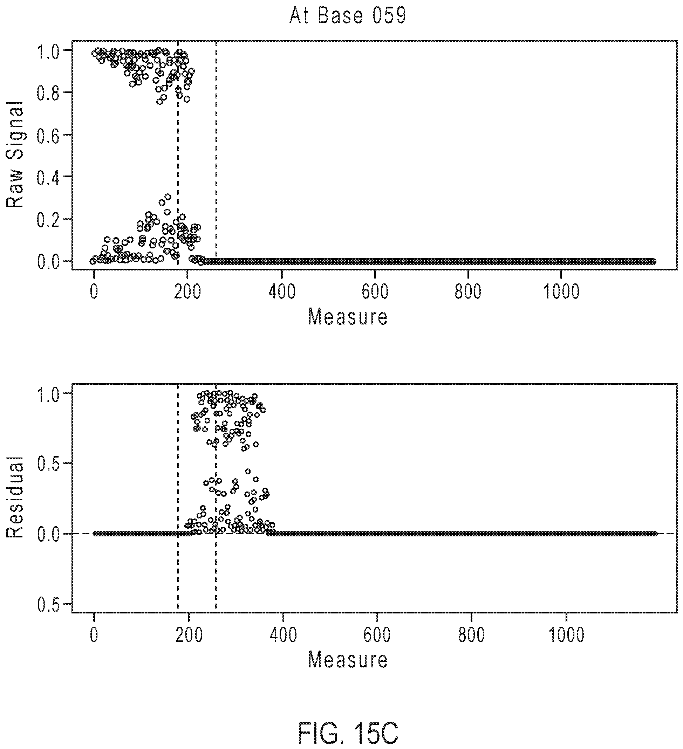

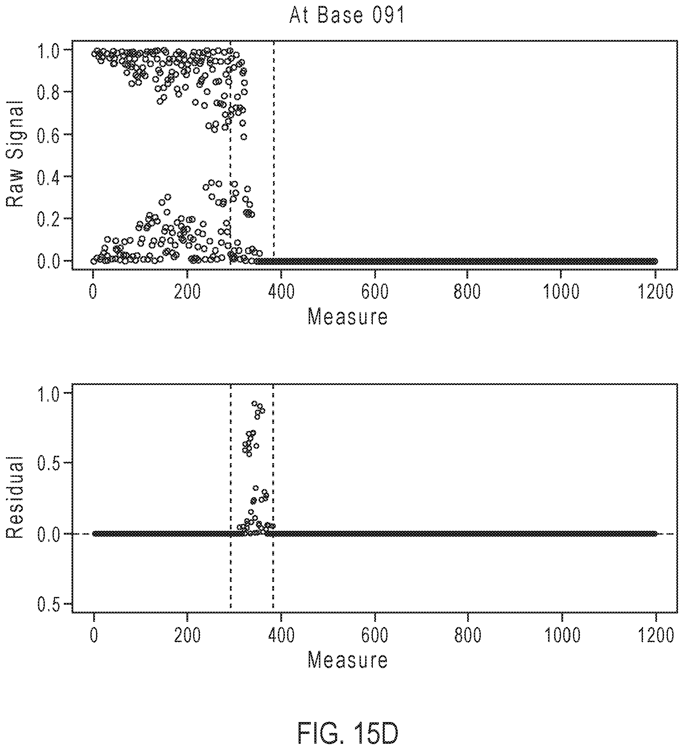

FIGS. 15A-15D illustrate exemplary simulation data corresponding to partially base-called simulated sequences for the sequencing reaction steps of FIG. 12A, according to an embodiment of the present disclosure. The top row of each of FIGS 15A-15D depicted by reference numeral 1510 shows the predicted signal as obtained through the called sequence and the simulation framework, whereas the bottom row 1520 shows a simulated measured signal that is not yet base-called.

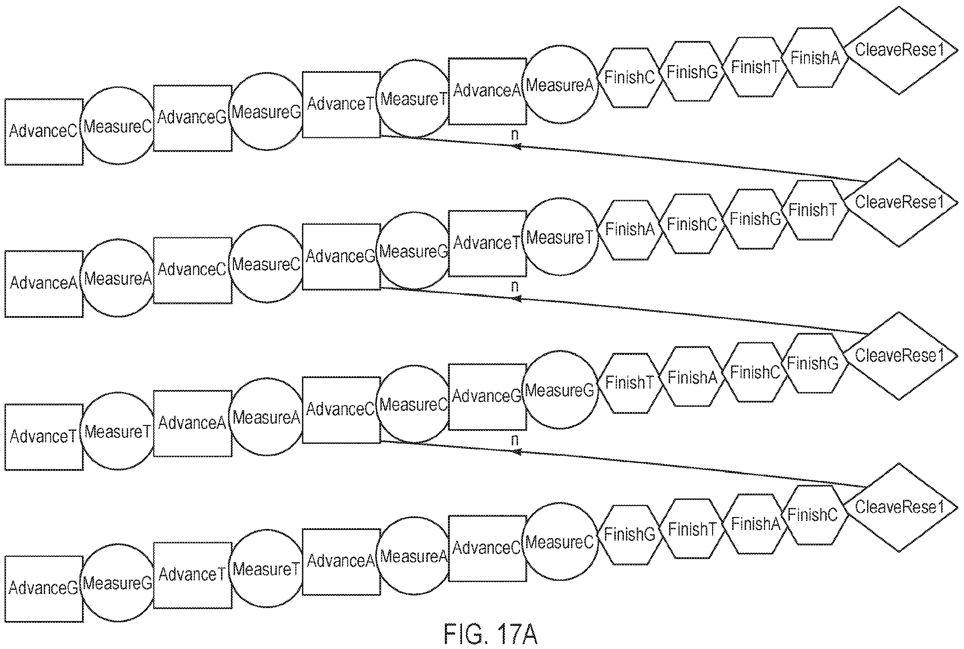

FIG. 17A illustrates sequencing reaction steps comprising advance and measure steps for four different terminating nucleotides for every finish and cleave/reset step per sequence, according to yet another exemplary embodiment of the present disclosure. According to this embodiment, four dNTPs (i.e. a "quad") are individually advanced and measured prior to the quad being finished and reset/cleaved. The exemplary nucleotide flow order in FIG. 17A may be represented as "GTAC, TACG, ACGT, CGTA" with a measurement step in between each advance, and finish and cleave/reset steps in between each pair of nucleotides. Although not shown herein, a wash step is optionally added at any point in the cycle; for example, subsequent to each cleave/reset step.

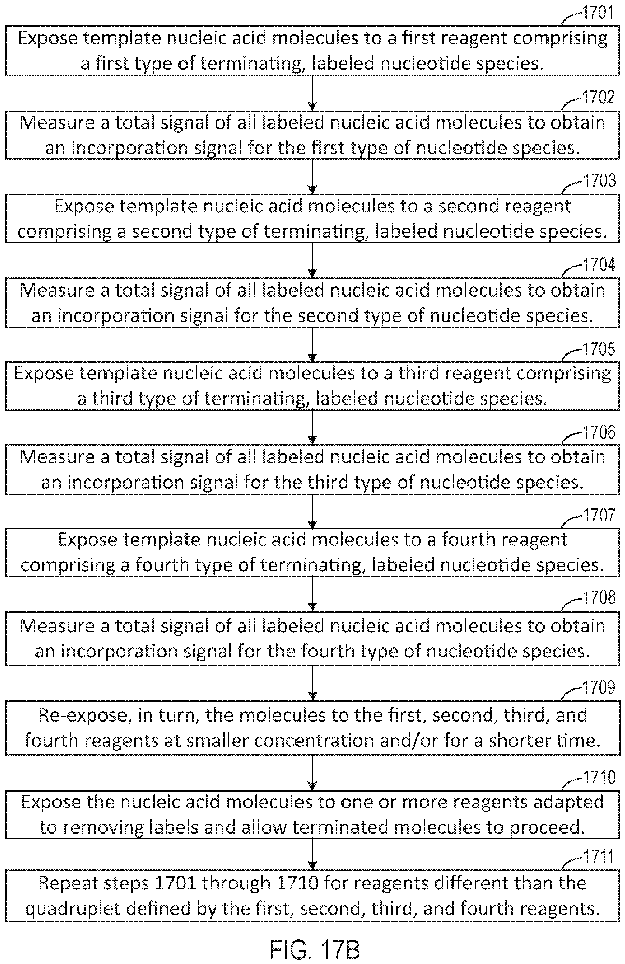

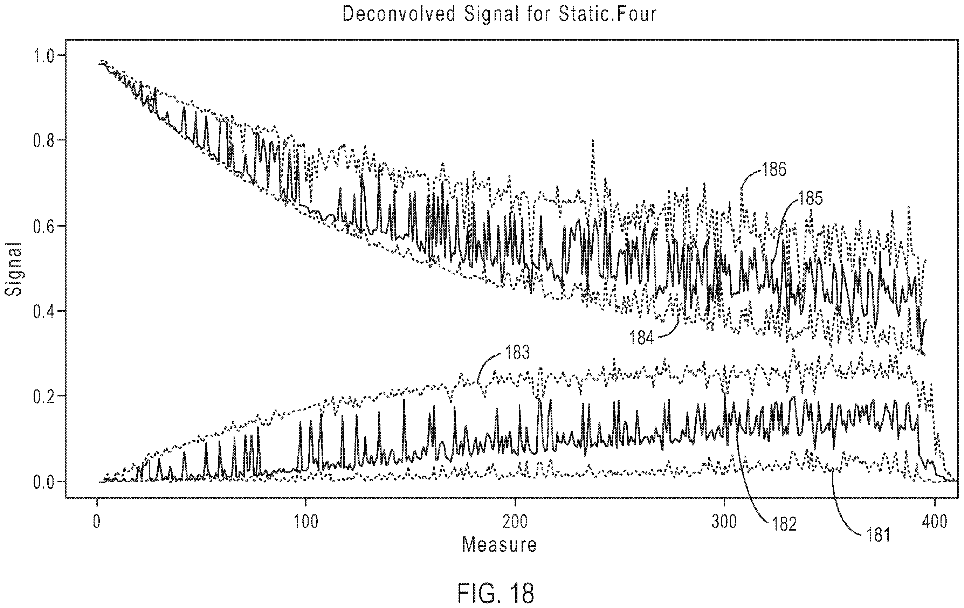

FIG. 17B is a flow chart illustrating a method for performing a nucleotide flow based on the sequencing reaction steps of FIG. 17A, according to an embodiment of the present disclosure. At 1701, an advance step exposes a collection of template nucleic acid molecules intended to be sequenced to a first reagent comprising a first type of nucleotide or terminating nucleotide species. The first reagent may be labeled with a labeling reagent that is associated with a response, such as pH or light, that can be measured. At 1702, a total signal of all labeled nucleotides is measured at equilibrium, to obtain a signal representative of incorporation of the first type of nucleotide or terminating nucleotide species. Subsequently at 1703, another advance step exposes a collection of template nucleic acid molecules intended to be sequenced to a second reagent comprising a second type of nucleotide or terminating nucleotide species. The second reagent may be labeled with a labeling reagent that is associated with a response different from the first reagent, such as pH or light. At 1704, a total signal of all labeled nucleotides is measured at equilibrium, to obtain a signal representative of incorporation of the second type of nucleotide or terminating nucleotide species. Further, at 1705, another advance step exposes a collection of template nucleic acid molecules intended to be sequenced to a third reagent comprising a third type of nucleotide or terminating nucleotide species. The third reagent may be labeled with a labeling reagent that is associated with a response different from the first and second reagents, such as pH or light. At 1706, a total signal of all labeled nucleotides is measured at equilibrium, to obtain a signal representative of incorporation of the third type of nucleotide or terminating nucleotide species. Further, at 1707, a fourth advance step exposes a collection of template nucleic acid molecules intended to be sequenced to a fourth reagent comprising a fourth type of nucleotide or terminating nucleotide species. The fourth reagent may be labeled with a labeling reagent that is associated with a response different from the first, second, and third reagents, such as pH or light. At 1708, a total signal of all labeled nucleotides is measured at equilibrium, to obtain a signal representative of incorporation of the third type of nucleotide or terminating nucleotide species. Signal response curves corresponding to steps 1702, 1704, 1706, and 1708 are further illustrated below in FIG. 18.

Subsequently, at 1709, finish steps are performed to re-expose the template molecules respectively to the first, second, third, and fourth reagents at a smaller concentration and/or for a shorter duration. At 1710, cleave and reset steps are performed to expose the template(s) to cleaving agents to remove labels from the labeled molecules, and to allow the terminated molecules to proceed through the system. Finally, at step 1711, steps 1701-1710 are repeated for each of a plurality of reagents respectively comprising a quad of nucleotide / terminating nucleotide species that are correspondingly labeled and that are different from the quad comprising the first, second, third, and fourth nucleotides.

FIG. 18 illustrates exemplary simulation data corresponding to signal response curves for the sequencing reaction steps of FIG. 17A, according to an embodiment of the present disclosure. Signal response curves are depicted with signal intensity on the y-axis and the nth flow number (time) on the x-axis, with two triplet sets of plot lines illustrated, each of the triplet sets having a darker solid line (182, 185) in the middle between two lighter dotted lines (181, 183; 184, 186). The bottom triplet set of plot lines (181, 182, 183) show the signal from 0-mer events (non-incorporation); and the top triplet set of plot lines (184, 185, 186) show the signal from 1-mer or 2-mer incorporation events. Within each triplet set, the darker solid line in the middle (182, 185) represents the median signal, the lighter dotted line above (183, 186) represents the 25 percentile signal, and the lighter dotted line below (181, 184) represents the 75 percentile signal. As shown in FIG. 18, while the signal for the 1-mer/2-mer incorporation events degrades as the sequencing read progresses, the signal produced by non-incorporation 0-mer events (e.g., the background signal) increases as the sequencing read progresses. Thus, at later portions of the sequencing read, the signal resolution diminishes and it becomes more difficult to distinguish the 0-mer events from 1-mer/2-mer events. As explained above, the accumulated effects of CF and/or IE events contribute to this degradation of signal quality

Notably, these accumulated effects are greater in this embodiment than in the nucleotide flows depicted in previous embodiments disclosed above, particularly when compared to the signal response curves simulated in FIG. 4. This difference may be attributed to the increased number of advance and measurement steps performed per finish/cleave/reset step. Nevertheless, as evidenced by FIG. 18, the signals are still sufficiently distinct from each other, owing to the phase-protecting sequence of nucleotides flowed in each successive advance step.

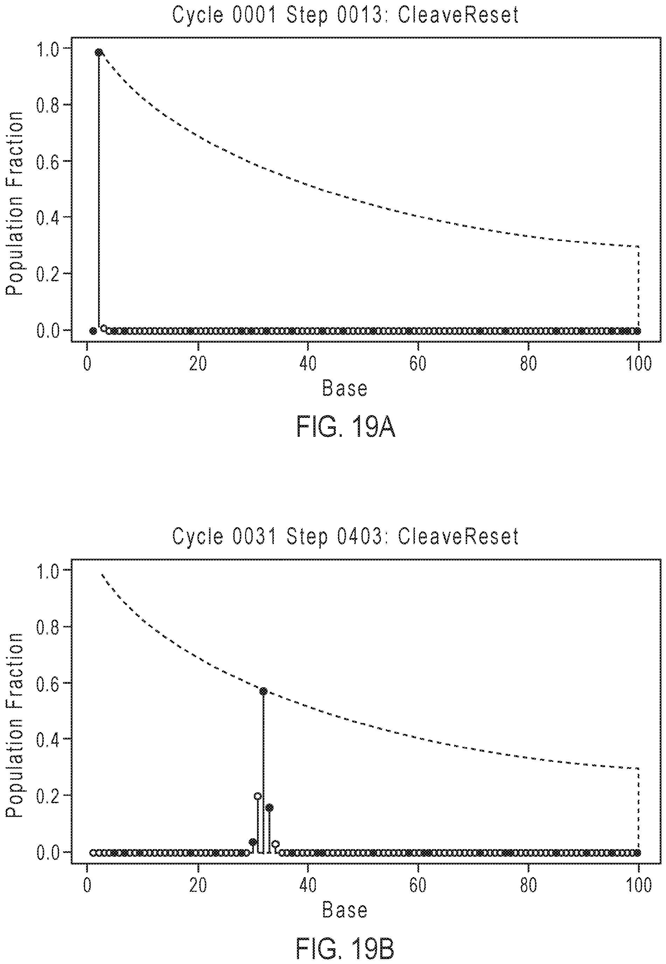

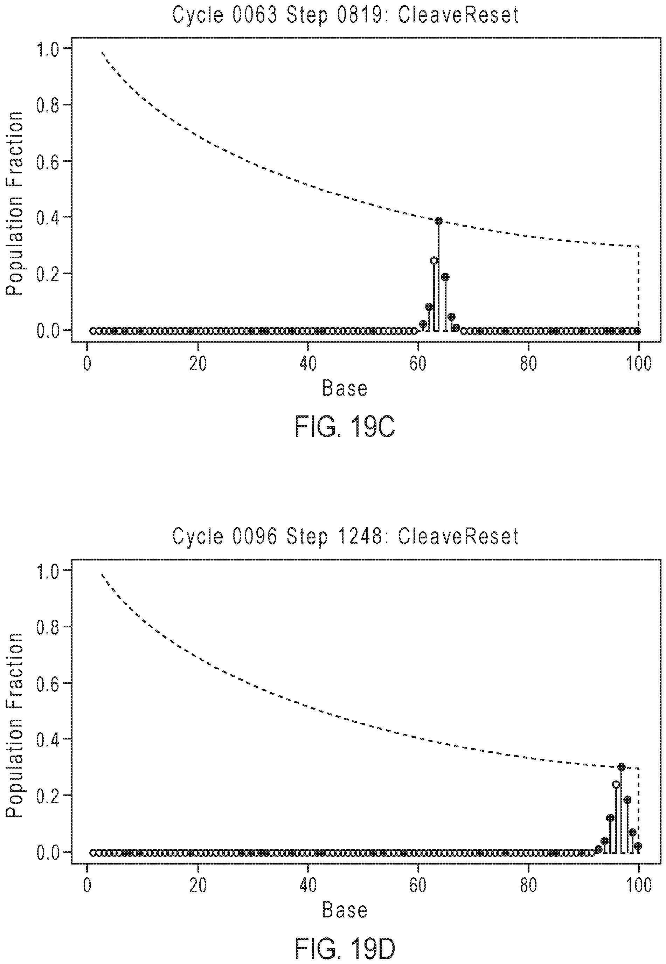

FIGS. 19A-19D illustrate exemplary simulation data corresponding to template population evolution as sequencing progresses for the sequencing reaction steps of FIG. 17A, according to an embodiment of the present disclosure.

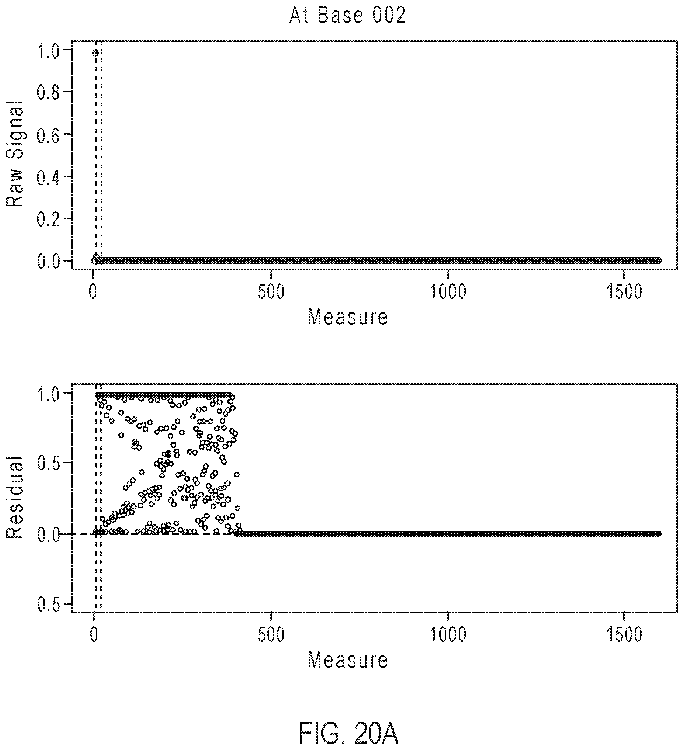

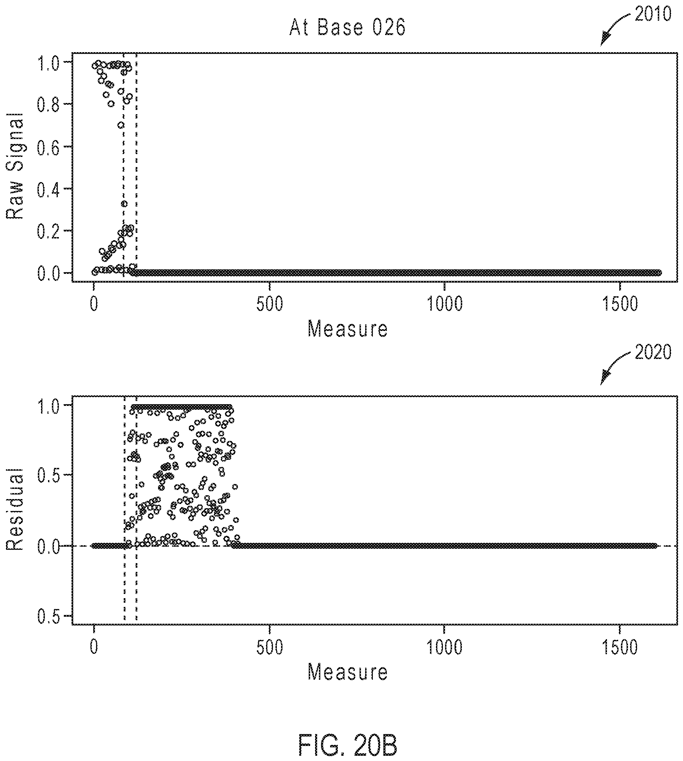

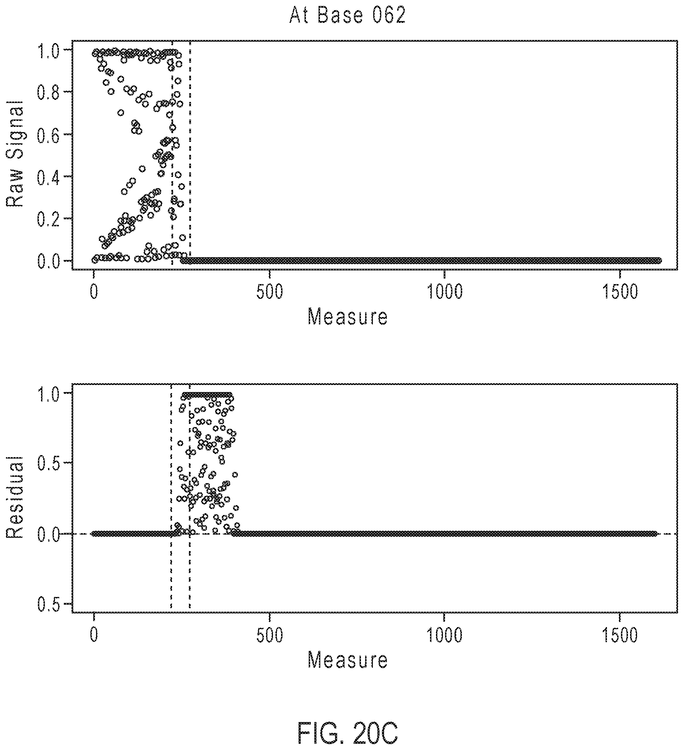

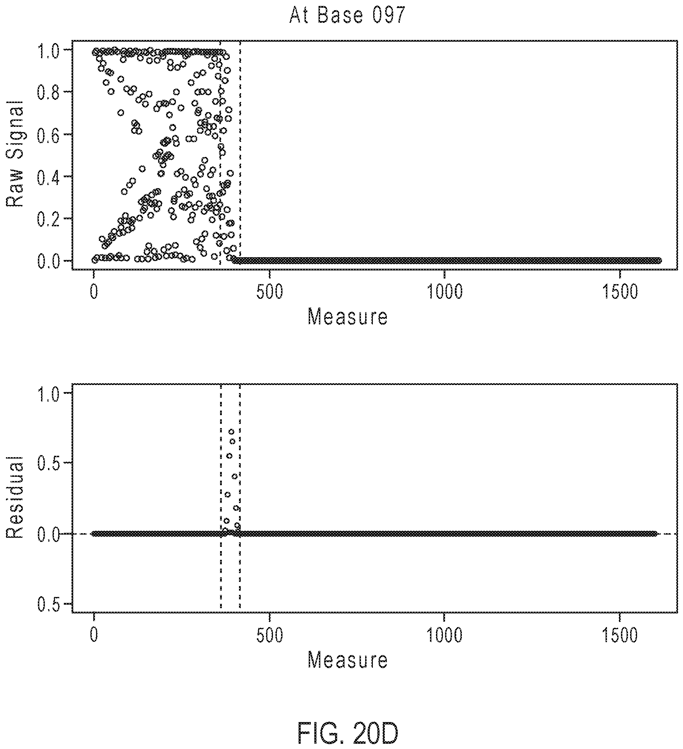

FIGS. 20A-20D illustrate exemplary simulation data corresponding to partially base-called simulated sequences for the sequencing reaction steps of FIG. 17A, according to an embodiment of the present disclosure. The top row of each of FIGS 20A-20D depicted by reference numeral 2010 shows the predicted signal as obtained through the called sequence and the simulation framework, whereas the bottom row 2020 shows a simulated measured signal that is not yet base-called.

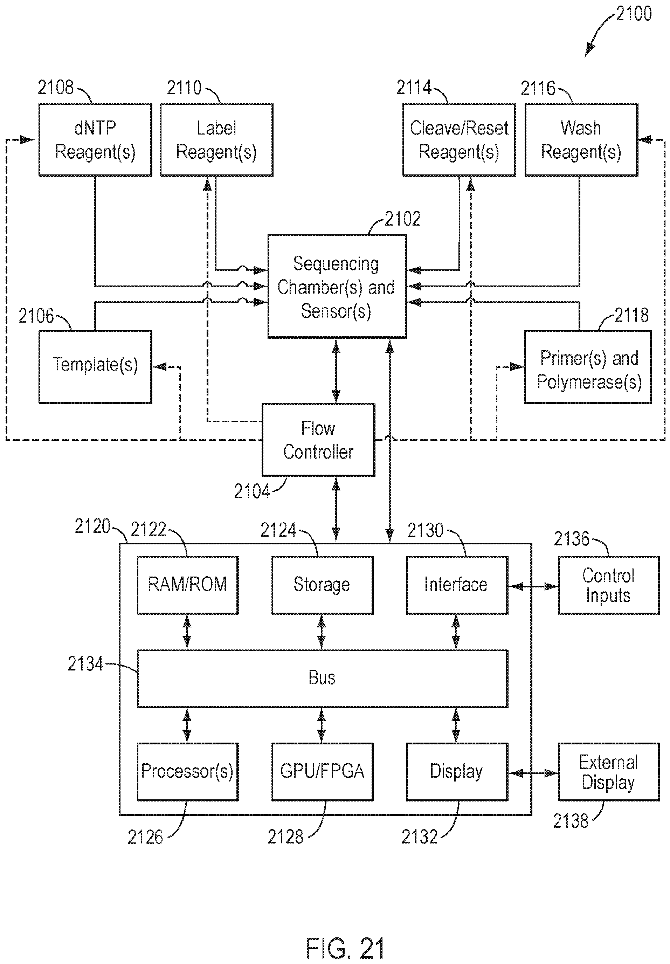

FIG. 21 is a schematic illustration of a system 2100 for nucleic acid sequencing, according to another exemplary embodiment of the present disclosure. The components of system 2100 are similar to those of system 100 illustrated in FIG. 1, with the exception that system 2100 does not include finisher reagents, and may utilize fewer tubes, solution reservoirs, and other components not depicted herein. For example, system 2100 includes a sequencing chamber 2102, a flow controller 2104, one or more template nucleic acids 2106, one or more nucleotide flow reagents 2108 comprising deoxynucleoside triphosphates (dNTPs), one or more label reagents 2110, one or more cleave/reset reagents 2114, one or more wash reagents 2116, one or more primers and/or polymerases 2118. System 2100 further comprises a computing device 2120 that includes memory 2122, storage 2124, one or more processors 2126, graphics processing unit (GPU) 2128, interface 2130, and display 2132 interconnected via bus 2134, as well as control inputs 2136 and external display 2138. Further, like system 2100, system 2100 is configured to perform a sequencing-by-synthesis process using termination chemistry ("termination sequencing-by-synthesis"). However, operations performed by system 2100 do not include a finishing step to incorporate additional molecules using the same base as was advanced in a prior advance step.

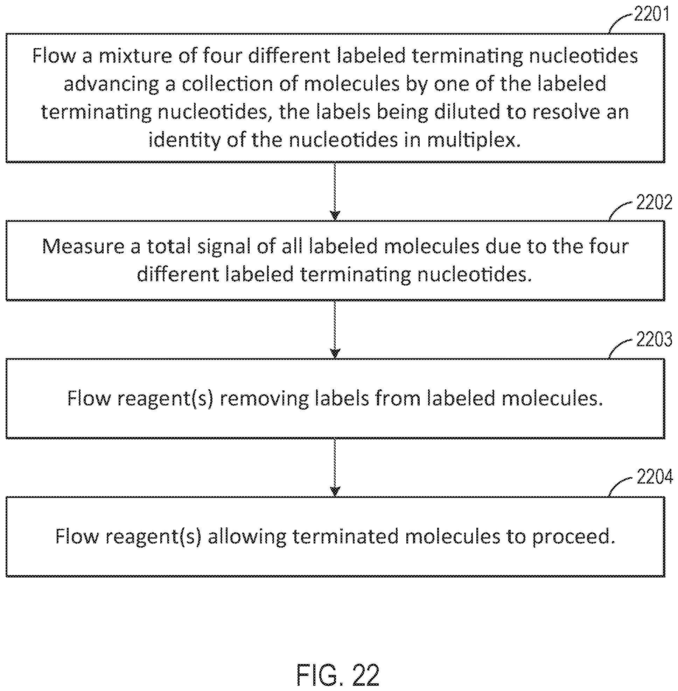

FIGS. 22-25 are flow charts illustrating methods for performing nucleotide flows based on various different sequencing reaction steps, according to embodiments of the present disclosure corresponding to system 2100 in FIG. 21. In these various exemplary embodiments, the above-described sequencing reaction steps may comprise cumulative measurements. In other words, a measurement performed after an advance step for any nucleotide will include measurements of both the nucleotide and the immediately preceding nucleotide that was advanced. Each subsequent measurement cumulatively includes signals for all preceding nucleotides that were advanced. In each of these embodiments, the component signals (i.e. individual signals associated with each terminating nucleotide) can be derived from the cumulative measurements, especially when the contribution of each component signal is linear or close to linear. These embodiments further minimize occurrence of finish, cleave, and reset steps that are more resource-intensive and time consuming. Further, phase error correction is maintained with increased numbers and combinations of nucleotides between each finish/cleave/reset step.

FIG. 22 is a flow chart illustrating a method for performing sequencing reaction steps using a cumulative measurement. At 2201, an advance step exposes a collection of template nucleic acid molecules intended to be sequenced to a mixture of four differently-labeled terminating nucleotides that are advanced by one of the labeled terminating nucleotides. Each label is diluted to enable resolution of an identity (i.e. component signal) of each labeled nucleotide in the multiplex. At 2202, a total signal of all labeled molecules is measured at equilibrium, to obtain a cumulative measurement, that may be processed to retrieve component signals corresponding to each labeled molecule. Subsequently, at 2203 and 2204, cleave and reset steps are performed to expose the template(s) to cleaving agents to remove labels from the labeled molecules, and to allow the terminated molecules to proceed through the system.

In another exemplary embodiment, sequencing reaction steps comprise advance and measure steps for two different terminating nucleotides for every finish and cleave/reset step per sequence, wherein the second measure step includes signals for both first and second terminating nucleotides.

In an exemplary embodiment, sequencing reaction steps comprise an advance step for advancing two nucleotides simultaneously, each of which is labeled differently, and individually measuring the signal associated with each nucleotide's label prior to finishing, resetting, and/or cleaving.

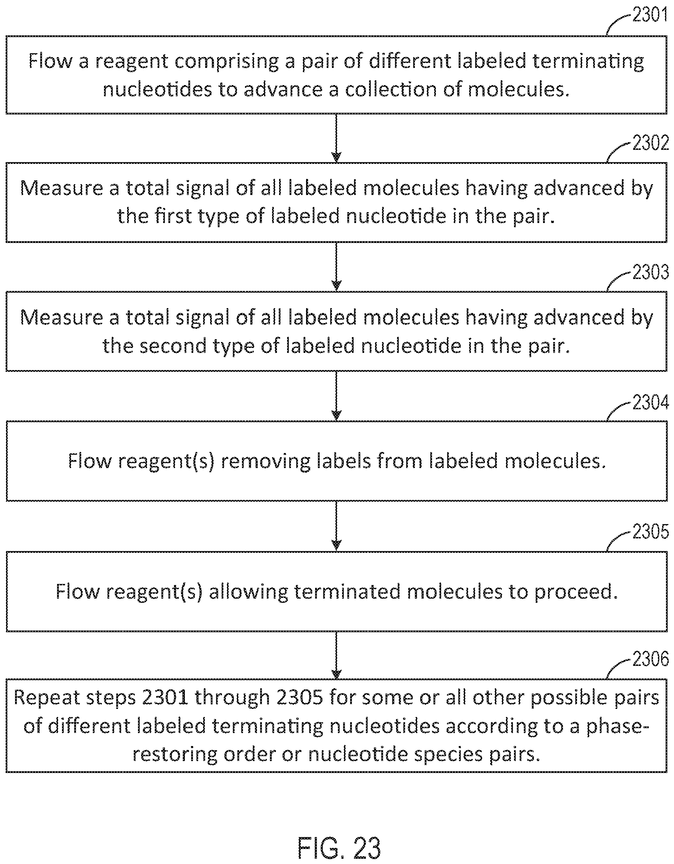

FIG. 23 is a flow chart illustrating a method for performing sequencing reaction steps using a cumulative measurement for a pair of differently-labeled terminating nucleotides for every finish and cleave/reset step per sequence. At 2301, an advance step exposes a collection of template nucleic acid molecules intended to be sequenced to a mixture (i.e. "duo") of two different terminating nucleotides. At 2302 and 2303, signals for each of the first and second terminating nucleotides are measured and, at 2304 and 2305, cleave and reset steps are performed to expose the template(s) to cleaving agents to remove labels from the labeled molecules, and to allow the terminated molecules to proceed through the system. Subsequently, at step 2306, steps 2301-2305 may be repeated for some or all other possible duos of differently-labeled terminating nucleotides according to a phase-restoring order, i.e. an order that mitigates phase errors, as described herein.

In another exemplary embodiment, sequencing reaction steps comprise advance and measure steps for three different terminating nucleotides for every finish and cleave/reset step per sequence, wherein the second measure step includes signals for both first and second terminating nucleotides, and the third measure step includes signals for first, second, and third terminating nucleotides.

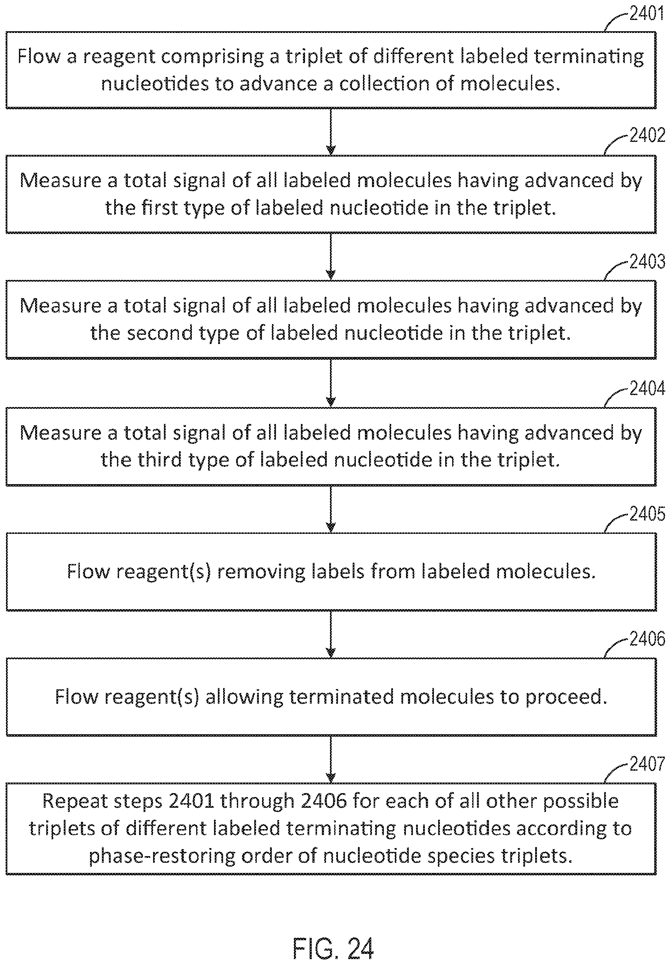

FIG. 24 is a flow chart illustrating a method for performing sequencing reaction steps using a cumulative measurement for a triplet of differently-labeled terminating nucleotides for every finish and cleave/reset step per sequence. At 2401, an advance step exposes a collection of template nucleic acid molecules intended to be sequenced to a mixture of three different terminating nucleotides. At 2402, 2403, and 2404, signals for each of the first, second, and third terminating nucleotides are measured and, at 2404 and 2405, cleave and reset steps are performed to expose the template(s) to cleaving agents to remove labels from the labeled molecules, and to allow the terminated molecules to proceed through the system. Subsequently, at step 2407, steps 2401-2406 may be repeated for some or all other possible triplets of differently-labeled terminating nucleotides according to a phase-restoring order, i.e. an order that mitigates phase errors, as described herein.

FIG. 25 is a flow chart illustrating a method for performing sequencing reaction steps using a cumulative measurement for a triplet of differently-labeled terminating nucleotides for every finish and cleave/reset step per sequence. At 2501, a first type of reagent from a first ordered triplet of differently-labeled terminating molecules is advanced over one or more template nucleic acid molecules intended to be sequenced and, at 2502, a total signal of all labeled molecules having advanced by the first type of labeled reagent is measured. Steps 2503-2506 repeat the advancing and measurement steps respectively for each of a second and third type of reagent and label thereof. Then, at 2507 and 2508, cleave and reset steps are performed to expose the template(s) to cleaving agents to remove labels from the labeled molecules, and to allow the terminated molecules to proceed through the system. Subsequently at 2509, the order of molecules in the triplet is changed according to a sequence or cycle, and at step 2509, steps 2501-2508 may be repeated for each respective order of the triplet.

In another exemplary embodiment, sequencing reaction steps comprise advance and measure steps for four different terminating nucleotides for every finish and cleave/reset step per sequence, wherein the second measure step includes signals for both first and second terminating nucleotides, the third measure step includes signals for first, second, and third terminating nucleotides, and the fourth measure step includes signals for first, second, third, and fourth terminating nucleotides.

In another exemplary embodiment utilizing the cumulative measurement described above, sequencing reaction steps comprise an advance step for advancing three terminating nucleotides simultaneously, each of which is labeled differently, performing a cumulative measurement associated with each label, cleaving a first label from a corresponding first terminating nucleotide, performing a cumulative measurement associated with the remaining two labels, cleaving a second label from a corresponding second terminating nucleotide, performing an individual measurement associated with the remaining third label, and cleaving the remaining third label prior to finishing, resetting, and/or final cleaving. This embodiment is particularly advantageous for systems where repeated cleave steps are faster than advance steps. Further, the described nucleotide flow order provides protection from phase errors.

Additional exemplary sequencing reaction steps described herein include advancing one or more terminating nucleotides in a sequence based on a type of label attached to each of said one or more terminating nucleotides, and measuring signals generated from the resulting incorporations. Advancing nucleotides by a label (or tag) rather than by a base of the nucleotides further reduces the system components required to sequence templates. For example, when a mixture comprising two or more differently-labeled terminating nucleotides is advanced, fewer solution reservoirs and tubes are needed. Similarly, cleave/reset steps for simultaneously cleaving multiple labels require fewer tubes and solution reservoirs. Further, complementary sets of nucleotides can be advanced in each mixture, thus enabling accurate measurement and minimizing the need for additional finishing steps.

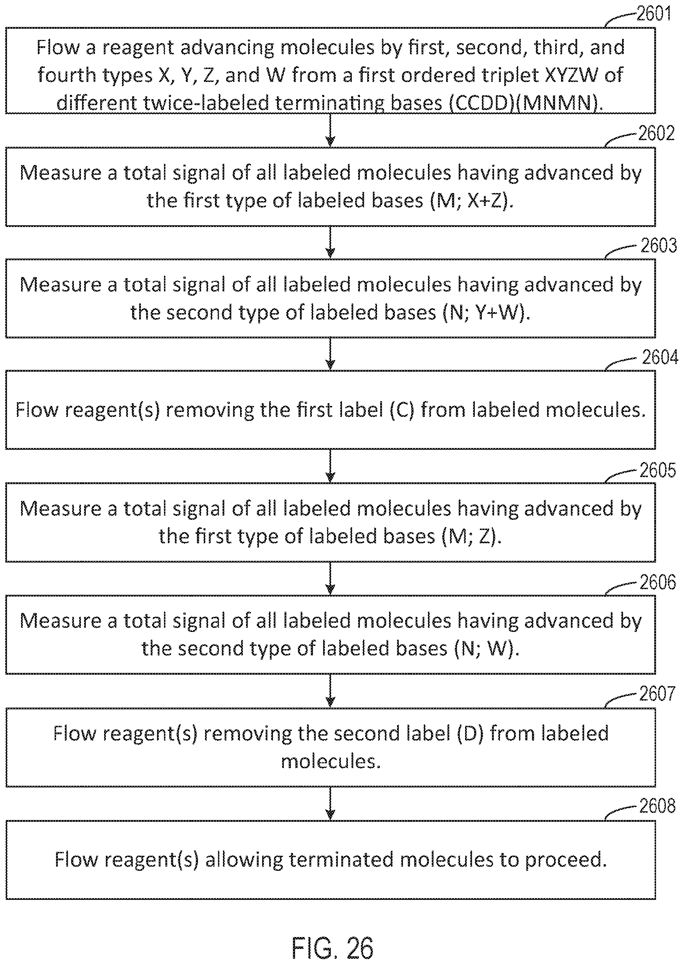

FIG. 26 is a flow chart illustrating a method for performing sequencing reaction steps by advancing twice-labeled nucleotides, according to an exemplary embodiment of the present disclosure. A twice-labeled nucleotide comprises a nucleotide that has more than one label attached to it to reduce the number of measuring steps that are necessary. Thus, four different twice-labeled nucleotides may be distinguished by having a red label, a green label, a red+green label, and no label. In this embodiment, four different combinations of two labels are used to distinguish four nucleotides. X=CM, Y=CN, Z=DM, W=DN, where M and N are the labels that are being measured and C and D are the linker molecules that bind M, N to the nucleotides. When C is removed (at 2604) then the labels CM and CN are removed from X,Y and those molecules will no longer show in subsequent measurements of M,N.

In particular, at 2601, an advance step exposes a collection of template nucleic acid molecules intended to be sequenced to a first ordered mixture of terminating nucleotides, each of which are labeled twice, i.e. with two different labels. For example, given nucleotides X, Y, Z, and W (with letters X, Y, Z, and W being representative of any one of nucleotide bases A, T, C, or G), nucleotide X may be labeled with label M with linker molecule C, nucleotide Y may be labeled with label N with linker molecule C, nucleotide Z may be labeled with label M with linker molecule D, and nucleotide W may be labeled with label N with linker molecule D. Thus, nucleotides X and Z share the same label M, nucleotides Y and W share the same label N, nucleotides X and Y share the same linker molecule C, and nucleotides Z and W share the same linker molecule D.

At 2602, a first total signal for molecules having advanced by a first type of labeled base is measured. For example, if a first signal corresponds to label M, then incorporations from nucleotides X and Z are obtained. Subsequently at 2603, a second total signal for molecules having advanced by a second type of labeled base is measured. For example, if a second signal corresponds to label N, then incorporations from nucleotides Y and W are obtained. At 2604, a reagent is flowed for removing linker molecule C from the labeled molecules. This results in removal of all M and N labels that were linked using linker molecule C. Thus, at 2605, a total signal is measured of all labeled molecules having advanced by base nucleotide Z with a label of M, i.e. nucleotides that are still labeled M while being linked by molecule D. Further, at 2606, a total signal is measured of all labeled molecules having advanced by base nucleotide W with a label of N, i.e. nucleotides that are still labeled N while being linked by molecule D.

Finally, at 2607, a reagent is flowed that removes linker molecule D from the labeled molecules, and at 2608, a finisher flow is provided to allow terminated molecules to proceed. As described herein, advancing nucleotides by a label (or tag) rather than by a base of the nucleotides reduces the system components required to sequence templates, such as solution reservoirs and tubes, and enables complementary sets of nucleotides to be advanced in each mixture, thus enabling accurate measurement and minimizing the need for additional finishing steps.

Further modifications and alternative embodiments will be apparent to those of ordinary skill in the art in view of the disclosure herein. For example, the systems and the methods may include additional components or steps that were omitted from the diagrams and description for clarity of operation. Accordingly, this description is to be construed as illustrative only and is for the purpose of teaching those skilled in the art the general manner of carrying out the present disclosure. It is to be understood that the various embodiments shown and described herein are to be taken as exemplary. Elements and materials, and arrangements of those elements and materials, may be substituted for those illustrated and described herein, parts and processes may be reversed, and certain features of the present teachings may be utilized independently, all as would be apparent to one skilled in the art after having the benefit of the description herein. Changes may be made in the elements described herein without departing from the spirit and scope of the present teachings and following claims.

It is to be understood that the particular examples and embodiments set forth herein are non-limiting, and modifications to structure, dimensions, materials, and methodologies may be made without departing from the scope of the present teachings.

Other embodiments in accordance with the present disclosure will be apparent to those skilled in the art from consideration of the specification and practice of the embodiments disclosed herein. It is intended that the specification and examples be considered as exemplary only, with the claims being entitled to their full breadth and scope, including equivalents.

## Claims

1. A method for nucleic acid sequencing, comprising:
disposing a plurality of template nucleic acid molecules in a plurality of defined spaces disposed on a sensor array, at least some of the plurality of template nucleic acid molecules having a sequencing primer and a polymerase operably bound therewith;
advancing a mixture of nucleotide species over the plurality of template nucleic acid molecules with the sequencing primer and the polymerase operably bound therewith;
measuring a cumulative signal generated by nucleotide incorporations resulting from advancing the mixture nucleotide species; and determining a contribution to the cumulative signal of each nucleotide species in the mixture of nucleotide species; and
cleaving a labeling reagent from one or more of the mixture of nucleotide species;
wherein the advancing of the mixture of nucleotides species and measuring signals generated therefrom are performed for different orders of mixture of nucleotide species prior to a subsequent cleaving.

2. The method of claim 1, wherein the cleaving reagent removes labeling reagents attached to each nucleotide species in the mixture of nucleotide species.

3. The method of claim 1 or claim 2, wherein the advancing a mixture of nucleotide species consists of advancing a pair of nucleotide species, or advancing a triplet of nucleotide species, or advancing a quad of nucleotide species.

4. The method of claim 3, wherein a pair of nucleotide species is advanced over the plurality of template nucleic acid molecules with the sequencing primer and the polymerase operably bound therewith and the advancing and measuring steps are repeated for different orders of nucleotide species per pair of nucleotide species prior to subsequent exposing steps.

5. The method of claim 3, wherein a triplet of nucleotide species is advanced over the plurality of template nucleic acid molecules with the sequencing primer and the polymerase operably bound therewith and the advancing and measuring steps are repeated for different orders of nucleotide species per triplet of nucleotide species prior to subsequent exposing steps.

6. The method of claim 5, wherein the method may further be repeated for alternating combinations of nucleotide species per triplet of nucleotide species.

7. The method of claim 3, wherein a quad of nucleotide species is advanced over the plurality of template nucleic acid molecules with the sequencing primer and the polymerase operably bound therewith and the advancing and measuring steps are repeated for different orders of nucleotide species per quad of nucleotide species prior to subsequent exposing steps.

8. The method of claim 7, wherein the method may further be repeated for alternating combinations of nucleotide species per quad of nucleotide species.

9. The method of any preceding claim, wherein the mixture of nucleotide species may be advanced in a phase-protecting flow order.

10. The method of any preceding claim, wherein each of the nucleotide species within the mixture is labeled differently.

11. The method of any preceding claim, wherein the mixture of nucleotide species contains terminating nucleotides.

12. The method of claim 10, comprising
a. advancing a pair of differently-labeled terminating nucleotides over the plurality of template nucleic acid molecules with the sequencing primer and the polymerase operably bound therewith,
b. measuring signals for each of the first and second terminating nucleotides;
c. performing cleave and reset steps expose the template(s) to cleaving agents to remove labels from the labeled molecules; and
d. repeating steps a. to c. for some or all other possible pairs of differently-labeled terminating nucleotides according to a phase-restoring order.

13. The method of claim 10, comprising
a. advancing a triplet of differently-labeled terminating nucleotides over the plurality of template nucleic acid molecules with the sequencing primer and the polymerase operably bound therewith,
b. measuring signals for each of the first, second and third terminating nucleotides;
c. performing cleave and reset steps expose the template(s) to cleaving agents to remove labels from the labeled molecules; and
d. repeating steps a. to c. for some or all other possible triplets of differently-labeled terminating nucleotides according to a phase-restoring order.

14. The method of claim 10, wherein four differently-labeled terminating nucleotides are advanced having four different combinations of two types of labels.

15. The method of claim 14, wherein the first type of label are the labels that are measured and the second type of label are the linker molecules that bind the labels to the nucleotides.
